# FASCICULE DE BREVET EUROPEEN

(11) **EP 2 389 158 B1**
(45) Date de publication et mention de la délivrance du brevet: **12.03.2014**
(21) Numéro de dépôt: 10701502.6
(22) Date de dépôt: 20.01.2010
(51) Int. Cl.: A61K 9/127, A61K 47/34, A61K 47/36, A61K 47/48, A61K 48/00

(54) **VECTEURS COMPRENANT UNE MACROMOLÉCULE ANIONIQUE ET UN LIPIDE CATIONIQUE POUR L'ADMINISTRATION DE PETITS ACIDES NUCLÉIQUES**
VEKTOREN, EINSCHLIESSLICH EINES ANIONISCHEN MAKROMOLEKÜLS UND EINES KATIONISCHEN LIPIDS ZUR ABGABE VON KLEINEN NUKLEINSÄUREN
VECTORS INCLUDING AN ANIONIC MACROMOLECULE AND A CATIONIC LIPID FOR DELIVERING SMALL NUCLEIC ACIDS

(30) Priorité: 20.01.2009 FR 0950336
(43) Date de publication de la demande: 30.11.2011
(73) Titulaire: Centre National de la Recherche Scientifique CNRS, 75016 Paris (FR); Université Paris Descartes, 75006 Paris (FR); INSERM - Institut National de la Santé et de la Recherche Médicale, 75013 Paris (FR)
(72) Inventeur: ESCRIOU, Virginie, F-94800 Villejuif (FR); BIGEY, Pascal, F-75020 Paris (FR); SCHERMAN, Daniel, F-75012 Paris (FR)
(74) Mandataire: Regimbeau
(86) Numéro de dépôt international: PCT/EP2010/050635
(87) Numéro de publication internationale: WO 2010/084129

(56) Documents cités:
- EP-A- 1 938 802
- WO-A-03/095641
- FR-A- 2 858 628
- MAROUN KHOURY SR ET AL: "EFFICIENT DELIVERY OF SMALL INTERFERING RNA TARGETING PRO-INFLAMMATORY CYTOKINES IN EXPERIMENTAL ARTHRITIS" ARTHRITIS AND RHEUMATISM, LIPPINCOTT, PHILADELPHIA, US, vol. 54, no. 9 SUPPL. S, 1 septembre 2006 (2006-09-01), page S174, XP009083272 ISSN: 0004-3591
- RHINN H ET AL: "How to make siRNA lipoplexes efficient? Add a DNA cargo" BIOCHIMICA ET BIOPHYSICA ACTA - GENERAL SUBJECTS, ELSEVIER SCIENCE PUBLISHERS, NL, vol. 1790, no. 4, 1 avril 2009 (2009-04-01), pages 219-230, XP025972957 ISSN: 0304-4165 [extrait le 2009-01-22]

## Description

La présente invention se rapporte à de nouveaux vecteurs pour l'administration intracellulaire d'acides nucléiques de petite taille, tels que des acides nucléiques capables de moduler une fonction protéique, et leur utilisation en thérapie génique.

De nombreuses maladies sont associées à un défaut d'expression et/ou une expression anormale, c'est-à-dire déficiente ou excessive, d'un ou plusieurs acides nucléiques. La thérapie génique a pour principal objectif de corriger ce type d'anomalies génétiques par le biais de l'expression cellulaire *in vivo* ou *in vitro* de gènes clonés.

De nombreux acides nucléiques de petite taille, tels que les ARN interférants (miRNA, siRNA, ...) sont capables de contrôler l'expression d'une protéine de manière spécifique en agissant sur l'ARNm de la protéine en question. Ils recrutent des complexes protéiques qui détruisent l'ARNm, ce qui conduit à la perte de l'expression de cette protéine ainsi que de sa fonction. Le site d'action de ces petits acides nucléiques est le cytoplasme des cellules. Or, il est difficile pour un acide nucléique de franchir la membrane cellulaire sans qu'il soit associé à un vecteur. En l'absence d'un vecteur, on peut obtenir une faible pénétration à condition d'ajouter l'acide nucléique à des concentrations très importantes qui, en plus d'être coûteuses en produit, conduisent à une extinction non spécifique de toutes les protéines de la cellule ainsi qu'à une toxicité.

Les vecteurs couramment utilisés et les plus efficaces pour faire pénétrer des acides nucléiques dans le cytoplasme d'une cellule sont des molécules chimiques cationiques, de type lipide ou polymère, qui s'associent de part leur charge à l'acide nucléique et lui permettent d'entrer dans la cellule. Ces molécules sont utilisées très couramment pour faire pénétrer des acides nucléiques (ADN ou ARN, de petite ou grande taille) dans des cellules en culture ou des cellules de l'organisme. Très efficaces dans les cellules en culture, elles le sont moins dans l'organisme du fait de phénomènes de piégeage par les protéines et les cellules contenues dans le sang. Par ailleurs, il est à noter que des molécules d'acide nucléique de petite taille sont capables d'interagir avec un lipide cationique mais les complexes formés n'ont pas nécessairement les mêmes propriétés que les complexes formés avec de l'ADN de grande taille.

Le document WO03/095641 décrit un véhicule pour l'administration intracellulaire qui comprend un lipide cationique, un liposome cationique, un lipoplexe comprenant un lipide cationique et un acide nucléique, ou bien un polymère anionique en association avec un lipide cationique. Lorsque le véhicule comprend un polymère anionique en association avec un lipide cationique, le polymère anionique peut inclure un groupe réactif couplé au siRNA.

Il a été proposé d'associer une molécule d'acide nucléique au PEI-alginate (PATNAIK et al, 2006). L'acide alginique est utilisé pour masquer les charges de PEI (polyethylenimine, qui est un polymère cationique), et ce, indépendamment de la molécule d'acide nucléique (ADN-ARN) qui est par la suite associée. La finalité de l'ajout de l'acide alginique est donc différente de celle de la présente invention, puisque dans PATNAIK et al. (2006), il ne s'agit alors pas d'aider à la formation des complexes entre le lipide cationique et la molécule d'acide nucléique de petite taille en ajoutant une macromolécule.

Il a également été proposé d'utiliser un ADN double brin de grande taille (appelé ADN cargo), dont la seule fonction est d'aider à l'association du siRNA avec le vecteur (KHOURY et al., 2006). Cet ADN cargo est un ADN plasmidique, double brin, circulaire, superenroulé.

Cependant, l'ADNdb cargo porte des séquences nucléotidiques susceptibles d'être transcrites par la machinerie cellulaire de la cellule recevant le vecteur, ce qui n'est pas compatible avec un développement clinique de la préparation.

De plus, de tels vecteurs doivent être non toxiques et biodégradables tout en étant stables en présence de sérum, notamment pour un développement clinique.

Les Inventeurs ont résolu ce problème en mettant au point une composition comprenant un certain type de macromolécule anionique et un lipide cationique en tant que vecteur d'acide nucléique de petite taille. Une telle composition permet d'améliorer la vectorisation des acides nucléiques de petite taille tout en étant non toxique, biodégradable et stable en présence de sérum.

Une différence majeure entre l'ADNdb cargo et les polymères anioniques tels que l'acide alginique ou l'acide polyglutamique, est que l'ADNdb cargo possède une topologie très particulière : il s'agit d'un ADN plasmidique, double brin, circulaire, superenroulé, couramment utilisé en association avec des lipides cationiques. D'un autre côté, l'acide alginique est utilisé en général pour ses capacités à former des gels insolubles, et l'acide polyglutamique, en greffage sur des composés hydrophobes pour augmenter leur solubilité dans l'eau. Ces polymères ne possèdent pas de topologie particulière comme l'ADN plasmidique.

Or, il est bien connu de l'homme de l'art que les acides nucléiques, tels que les ADN plasmidiques, sont capables de s'associer à des lipides cationiques, mais ce n'est pas du tout le cas pour tout autre polymère anionique. D'autre part, les acides nucléiques sont souvent des macromolécules double brin relativement rigides, ce qui n'est pas forcément le cas des polymères anioniques en général. Ainsi, il n'était pas évident qu'un polymère anionique non nucléotidique soit capable de former des associations ternaires avec un lipide cationique et un acide nucléique, et ainsi de conduire à une augmentation de l'effet biologique des acides nucléiques de petite taille incorporés dans ces complexes ternaires. En effet, on aurait pu imaginer qu'il se forme uniquement un mélange de complexes binaires, constitués de lipides et d'acide nucléique d'une part et de lipides et de polymère d'autre part.

Ainsi, la présente invention se rapporte à une composition comprenant :
(1) une macromolécule anionique à l'exception des acides nucléiques,
(2) un lipide cationique, et
(3) un acide nucléique de taille inférieure ou égale à 200 nucléotides dans laquelle ladite molécule anionique, ledit liquide cationique et ledit acide nucléique sont associés de manière non covalente.

Dans la présente invention, on entend par « macromolécule anionique » une molécule de poids moléculaire élevé présentant une charge globale négative, à l'exception des acides nucléiques.

Dans le cadre de la présente invention, on entend par « acide nucléique » un polymère dont l'unité de base est le nucléotide, les différents nucléotides étant liés les uns aux autres par des liaisons phosphodiesters.

Une macromolécule résulte généralement de l'assemblage, notamment par des liaisons covalentes, d'un grand nombre de groupements chimiques semblables ou différents nommés motifs de répétition.

La macromolécule anionique selon la présente invention peut être un polymère ou peut être formée par autoassociation, via des liaisons hydrogènes, de monomères complémentaires.

De préférence, la macromolécule anionique selon la présente invention est choisie parmi les polysaccharides anioniques, les polypeptides anioniques, les polyélectrolytes synthétiques comme le sodium polystyrène sulfonate et le carboxymethyl cellulose (CMC), les polyphosphates, les polyosides comme le dextran.

De manière particulièrement préférée, la macromolécule anionique selon la présente invention est choisie parmi les polyphosphates, les polysaccharides anioniques et les polypeptides anioniques, encore plus préférentiellement parmi les polysaccharides anioniques et les polypeptides anioniques.

Tel que défini dans la présente invention, un « polysaccharide » est formé par des séquences de saccharides connectées entre elles par des liaisons glycosidiques, et un « polysaccharide anionique » est un polysaccharide présentant une charge globale négative.

Parmi les polysaccharides anioniques, on peut citer les carrhagénanes (polysaccharides sulfatés des algues rouges), les fucanes (polysaccharides sulfatés des algues brunes), les CarboxyMéthyl Dextrane Benzylamide Sulfonates ou CMDBS (polysaccharides synthétiques préparés à partir de dextrane par substitution statistique de fonctions hydroxyles par des fonctions chimiques carboxyméthyles, benzylamides, sulfonates et sulfates), et les héparanes sulfates (polysaccharides complexes, appartenant à la famille des glycosaminoglycanes).

Avantageusement le polysaccharidique anionique est l'acide alginique ou l'un de ses sels, tels que le sel de sodium (SIGMA ALDRICH). L'acide alginique (CAS : 9005-32-7) est un polysaccharide colloïdal extrait de diverses variétés d'algues brunes, en particulier de *Laminaria*. Il a pour monomères constitutifs de l'acide alpha-L-glucuronique et beta-D-mannuronique liés par paire selon des liaisons de type 1->4. L'acide alginique est constitué en moyenne de 200 unités de base d'acides uroniques. Son poids moléculaire est généralement compris entre 10 000 et 600 000 Daltons.

Tel que défini dans la présente invention, un « polypeptide » est un polymère linéaire composé d'acides aminés liés par des liaisons covalentes, et un « polypeptide anionique » est un polypeptide présentant une charge globale négative.

Le polypeptide anionique peut être tout polypeptide naturel ou synthétique anionique. Il peut être un polypeptide mixte polyglutamique-polyaspartique, ou un polypeptide comportant une charge globale négative provenant d'acides aminés naturels (acide aspartique, acide glutamique) ou non naturels.

Avantageusement, le polypeptidique anionique est l'acide polyglutamique ou l'un de ses sels, tels que le sel de sodium (SIGMA ALDRICH) ou l'acide polyaspartique ou l'un de ses sels, ou encore le sulfate de dextran ou l'un de ses sels, tels que le sel de sodium (tel que celui disponible chez SIGMA ALDRICH) ou encore le carboxymethyl cellulose ou l'un de ses sels, tels que le sel de sodium (tel que celui disponible chez SIGMA ALDRICH), ou encore l'acide polyacrylique ou l'un de ses sels, tels que le sel de sodium (tel que celui disponible chez Fluka).

L'acide polyglutamique est un polymère soluble dans l'eau, biodégradable, comestible et non toxique.

De préférence, la macromolécule anionique a un poids moléculaire moyen compris entre 1000 et 1000000 Da, préférentiellement compris entre 1000 et 100000 Da encore plus préférentiellement compris entre 4300 et 56000 Da. Le poids moléculaire moyen dépendra du type de polymère et de sa voie de préparation (synthèse chimique ou purification à partir de matériel biologique).

Dans la présente invention, on entend par « lipide cationique », un lipide ayant une charge globale positive. Un lipide cationique est composé d'une tête polaire cationique et d'une entité hydrophobe de type chaîne lipidique ou cholestérol.

De préférence, le lipide cationique est choisi parmi :
- Les lipopolyamines, telles que la 2-{3-[Bis-(3-amino-propyl)-amino]-propylamino}-N-ditetradecyl carbamoyl methyl-acetamide (composé RPR209120), la 2-{3-[3-(3-Amino-propylamino)-propylamino]-propylamino}-N,N-dioctadecyl-acetamide (RPR120535) (Byk et al, J. Med. Chem., 41, 224-235, 1998) ou le 2,3-dioleoyloxy- N-[2(sperminecarboxamido)ethyl]-N,N-dimethyl- 1 -propane-aminium-trifluoracetate (DOSPA), le dioctadécylamine-glycine-- spermine (DOGS), la dipalmitylphosphatidylethanolamine 5-carboxyspermylamide (DPPES), ainsi que toutes les lipopolyamines décrites p.2, L.27 à p.4, L.19 dans la demande internationale WO 97/18185 telle que publiée, avantageusement les lipolyamines de formule choisie parmi les formules III à XII p.5, L.1 à p.7, L.9 de la demande WO 97/18185 telle que publiée, et de manière encore plus avantageuse p.8, L.29 à p.14, L.15 de la demande WO 97/18185 telle que publiée.
- Les ammoniums quartenaires tels que le bromure de 1,2-dimyristoyloxypropyl-3-diméthyl-hydroxy éthyl ammonium bromide (DMRIE) N-(2,3-dioleyloxypropyl]-N,N,N-trimethylammonium chloride (DOTMA), le 1,2-dioleoyloxypropyl-N,N,N-trimethylammonium chloride (DOTAP), la dimethyldioctadecylammonium bromide (DDAB), ou la 1,2-dioleyloxypropyl-3- dimethylhydroxyethylammonium bromide (DORIE), et
- Les lipides comportant des têtes cationiques de type guanidine (BGTC) ou imidazole (DOTIM).

De préférence, le lipide cationique est formulé soit sous forme de micelles, soit sous forme de liposomes par association avec un lipide neutre tel que la dioleyl phosphatidyl éthanolamine (DOPE achetée chez AVANTI POLAR LIPIDS), le Cholestérol, ou le (F8E11)(C16)OPE (encore nommé *N*'-(*rac*-1-[11-(*F*-octyl)undec-10-enyl]-2-(hexadecyl)glycero-3-phosphoethanoyl)-sperminecarboxamide), de préférence la dioleyl phosphatidyl éthanolamine.

Le lipide cationique peut également être formulé en réalisant des injections éthanoliques ou en le préparant avec un détergent.

Ladite composition selon l'invention peut également comprendre du polyéthylèneglycol (PEG), notamment lorsque ladite composition est utilisée pour des applications *in vivo*, tels que pour faire pénétrer des acides nucléiques dans des cellules d'un organisme.

La présence de PEG dans les compositions selon l'invention permet notamment d'augmenter la stabilité colloïdale des lipoplexes selon l'invention.

La composition selon l'invention peut comprendre entre 0,1 et 10%, en particulier entre 1 et 5% en poids de PEG par rapport au poids total de lipides dans la composition selon l'invention.

De préférence, les micelles sont préparées par ajout d'eau ou de tampon au lipide cationique, puis, après agitation vigoureuse le mélange est soumis à plusieurs cycles de congélation/chauffage à 55°C jusqu'à l'obtention d'une suspension claire de micelles. A titre de préparation liposomale, on peut citer la LipofectAmine (Gibco BRL), qui est préparée à partir de la DOSPA (lipopolyamine) et de la DOPE (lipide neutre). Avantageusement, le lipide cationique est présent en une quantité comprise entre 1 et 20 nmoles par µg de mélange d'acide nucléique et de macromolécule anionique, encore plus avantageusement entre 3 à 8 nmoles par µg de mélange d'acide nucléique et de macromolécule anionique, et de façon davantage préférée entre 4 et 6 nmoles par µg de mélange d'acide nucléique et de macromolécule anionique.

Dans le cadre de la présente invention, on entend par « acide nucléique de taille inférieure ou égale à 200 nucléotides» ou « acide nucléique de petite taille » un acide désoxyribonucléique (ADN), un acide ribonucléique (ARN) simple ou double brin, ou des séquences hybrides ADN/ARN de taille inférieure ou égale à 200 nucléotides. Il peut s'agir de séquences d'origine naturelle ou artificielle. Ils peuvent également être obtenus par une modification chimique au niveau de leurs parties sucres, de leurs parties nucléobases ou de leur squelette internucléotidique. Parmi les modifications avantageuses dans les parties sucres, on peut notamment citer les modifications intervenant en position 2' du ribose, telles que les modifications 2'-deoxy, 2'-fluoro, 2'-amino, 2'-thio, ou 2'-O-alkyl, en particulier 2'-O-méthyle, au lieu du groupement 2'-OH normal sur les ribonucléotides, ou encore la présence d'un pont méthylène entre les positions 2' et 4' du ribose (LNA). Concernant les nucléobases, il est possible d'utiliser des bases modifiées, telles que notamment la 5-bromo-uridine, la 5-iodo-uridine, la N³-méthyl-uridine, une 2,6-diaminopurine (DAP), la 5-méthyl-2'-deoxyCytidine, la 5-(1-propynyl)-2'-deoxy-Uridine (pdU), la 5-(1-propynyl)-2'-deoxyCytidine (pdC), ou des bases conjuguées avec du cholestérol. Enfin, des modifications avantageuses du squelette internucléotidique comprennent le remplacement de groupements phosphodiesters de ce squelette par des groupements phosphorothioate, méthylphosphonate, phosphorodiamidate, ou l'utilisation d'un squelette composé d'unités de N-(2-aminoethyl)-glycine liées par des liaisons peptidiques (PNA, Peptide Nucleic Acid). Les différentes modifications (base, sucre, squelette) peuvent bien entendu être combinées pour donner des acides nucléiques modifiés de type morpholino (bases fixées sur un cycle morpholine et liées par des groupes phosphorodiamidate) ou PNA (bases fixées sur des unités de N-(2-aminoethyl)-glycine liées par des liaisons peptidiques).

De préférence, l'acide nucléique de taille inférieure ou égale à 200 nucléotides est un acide nucléique capable de moduler une fonction protéique.

Dans la présente invention, on entend par « acide nucléique capable de moduler une fonction protéique » un acide nucléique qui (1) soit augmente et/ou restaure, au moins partiellement, (2) soit inhibe et/ou retarde et/ou bloque une fonction protéique.

De tels acides nucléiques sont bien connus de l'homme du métier. Il s'agit par exemple des acides nucléiques agissant sur l'expression d'un gène, tels que les oligonucléotides antisens ou les ARN interférants (miRNA, siRNA, ...). Il peut s'agir également des oligonucléotides pour le saut d'exon ou pour la modification d'épissage alternatif. Il peut encore s'agir des aptamères.

On entend par « oligonucléotide antisens » une séquence nucléotidique complémentaire d'une autre sequence nucléotidique, telle qu'une séquence d'ARNm, qui se fixe à cette dernière et empêche sa traduction en protéine correspondante, ou pénètre dans le noyau, se fixe à un brin d'ADN, et forme une triple hélice d'ADN, non transcriptible en ARN.

On entend par « ARN interférant » un acide ribonucléique simple ou double brin, qui interfère avec un ARN messager spécifique conduisant à sa dégradation et à la diminution de sa traduction en protéine. Il s'agit notamment des petits ARN interférants (« siRNA ») et des microARN (« miRNA »). (Wu et al, 2008).

On entend par « oligonucléotide pour le saut d'exon » une séquence nucléotidique spécifique d'un ARN pré-messager et capable d'interagir avec la machinerie d'épissage de sorte que l'épissage produise un ARN messager tronqué d'un ou plusieurs exons mais dont le cadre de lecture global n'est pas modifié, ce qui permet d'obtenir une protéine tronquée mais partiellement fonctionnelle (Van Ommen et al; 2008).

On entend par « aptamère » un oligonucléotide modifié sélectionné à partir d'une banque aléatoire de séquences en fonction de son affinité pour un ligand biologique en vue de son utilisation thérapeutique. Les aptamères se différencient des autres séquences d'acides nucléiques par leur capacité à se replier en une structure tertiaire pour créer une poche de liaison permettant d'interagir précisément et spécifiquement avec la cible. Les aptamères possèdent de nombreuses applications thérapeutiques (Kaur et Roy, 2008).

On entend par « acide nucléique pour la modification d'épissage alternatif » un acide nucléique permettant de modifier l'ARN pré-messager dans le but d'obtenir l'expression d'une protéine modifiée.

De préférence, l'acide nucléique capable de moduler une fonction protéique est choisi parmi un oligonucléotide antisens, un oligonucléotide pour le saut d'exon ou un oligonucléotide pour la modification d'épissage alternatif ou un ARN interférant, encore plus préférentiellement un ARN interférant, et/ou les formes modifiées de ces acides nucléiques, telles que les formes morpholino, phosphorothioate, phosphoroamidate et 2'Omethyl.

De manière encore plus préférée, ledit ARN interférant est un petit ARN interférant (« siRNA ») ou un microARN (« miRNA »), de manière préférée entre toutes un petit ARN interférant.

De préférence, l'acide nucléique a une taille inférieure à 200 nucléotides, de manière encore préférée inférieure à 100 nucléotides, de manière particulièrement préférée inférieure à 50 nucléotides, et de manière encore plus préférée inférieure à 30 nucléotides. De manière préférée entre toutes, l'acide nucléique a une taille comprise entre 20 et 25 nucléotides.

De préférence, lorsque l'acide nucléique de petite taille est un petit ARN interférant, celui-ci a une taille comprise entre 19 et 25 nucléotides.

De préférence, lorsque l'acide nucléique de petite taille est un microARN, celui-ci a une taille comprise entre 20 et 25 nucléotides.

De préférence, lorsque l'acide nucléique de petite taille est un oligonucléotide antisens, celui-ci a une taille comprise entre 13 et 25 nucléotides.

De préférence, lorsque l'acide nucléique de petite taille est un oligonucléotide pour le saut d'exon, celui-ci a une taille comprise entre 17 et 31 nucléotides.

De préférence, lorsque l'acide nucléique de petite taille est un oligonucléotide pour la modification d'épissage alternatif, celui-ci a une taille comprise entre 15 et 30 nucléotides.

De préférence, lorsque l'acide nucléique de petite taille est un aptamère, celui-ci a une taille comprise entre 20 et 80 nucléotides.

De préférence, la concentration en acide nucléique dans la composition selon la présente invention pour une utilisation *in vitro* est comprise entre 1 et 100 nM, encore plus préférentiellement entre 5 et 50 nM, et de manière préférée entre toutes entre 15 et 25 nM.

De préférence, la concentration en acide nucléique dans la composition selon la présente invention pour une utilisation *in vivo* est comprise entre 0,1 à 2,5 mg/kg, encore plus préférentiellement entre 0,3 et 1 mg/kg, et de manière préférée entre toutes entre 0,4 et 0,6 mg/kg.

Une composition préférée selon la présente invention est telle que la macromolécule anionique est l'acide alginique, le lipide cationique est une lipopolyamine, de préférence le 2-{3-[Bis-(3-amino-propyl)-amino]-propylamino}-N-ditetradecyl carbamoyl methyl-acetamide (RPR209120), et l'acide nucléique de taille inférieure ou égale à 200 nucléotides est un acide nucléique capable de moduler une fonction protéique, ledit acide nucléique étant de préférence choisi parmi un siRNA ou un oligonucléotide pour le saut d'exon ou pour la modification de l'épissage alternatif, ledit composé RPR209120 étant avantageusement formulé en liposome par association avec un lipide neutre tel que la DOPE.

De préférence, la composition selon l'invention est telle que la macromolécule anionique est l'acide alginique, le lipide cationique est le composé RPR209120 et l'acide nucléique de taille inférieure ou égale à 200 nucléotides est un petit ARN interférant, ledit composé RPR209120 étant avantageusement formulé en liposome par association avec un lipide neutre tel que la DOPE.

Une autre composition préférée selon la présente invention est telle que la macromolécule anionique est l'acide polyglutamique, le lipide cationique est une lipopolyamine, de préférence le composé RPR209120, et l'acide nucléique de taille inférieure ou égale à 200 nucléotides est un acide nucléique capable de moduler une fonction protéique, ledit acide nucléique étant de préférence choisi parmi un siRNA ou un oligonucléotide pour le saut d'exon ou pour la modification de l'épissage alternatif, ledit composé RPR209120 étant avantageusement formulé en liposome par association avec un lipide neutre tel que la DOPE.

De préférence, la composition selon la présente invention est telle que la macromolécule anionique est l'acide polyglutamique, le lipide cationique est le composé RPR209120 et l'acide nucléique de taille inférieure ou égale à 200 nucléotides est un petit ARN interférant, ledit composé RPR209120 étant avantageusement formulé en liposome par association avec un lipide neutre tel que la DOPE.

Une autre composition préférée selon la présente invention est telle que la macromolécule anionique est le polyphosphate, le lipide cationique est une lipopolyamine, de préférence le composé RPR209120, et l'acide nucléique de taille inférieure ou égale à 200 nucléotides est un acide nucléique capable de moduler une fonction protéique, ledit acide nucléique étant de préférence choisi parmi un siRNA ou un oligonucléotide pour le saut d'exon ou pour la modification de l'épissage alternatif, ledit composé RPR209120 étant avantageusement formulé en liposome par association avec un lipide neutre tel que la DOPE.

De préférence, la macromolécule anionique est pré-associée à l'acide nucléique. On entend par « macromolécule anionique pré-associée » le fait que l'acide nucléique soit mis au contact de la macromolécule anionique préalablement à la mise en contact avec le lipide cationique, ce qui permet la formation d'un mélange acide nucléique/macromolécule anionique. De préférence, le rapport macromolécule anionique/acide nucléique est compris entre 0,5 et 4 (poids/poids), de préférence compris entre 1 et 2, de manière encore préférée égale à 1.

Le mélange ainsi formé est ensuite mis en contact avec le lipide cationique, ce qui permet d'obtenir le complexe macromolécule anionique/acide nucléique/lipide cationique. De préférence, la mise en contact du mélange avec le lipide cationique est effectuée volume à volume. Dans le complexe ainsi obtenu, ladite macromolécule anionique, ledit lipide cationique et ledit acide nucléique sont associés de manière non covalente.

Ainsi, la composition selon l'invention comprend :
(1) une macromolécule anionique à l'exception des acides nucléiques,
(2) un lipide cationique, et
(3) un acide nucléique de taille inférieure ou égale à 200 nucléotides ;
dans laquelle ladite macromolécule anionique, ledit lipide cationique et ledit acide nucléique sont associés de manière non covalente.

De préférence, la composition selon la présente invention est présente dans un milieu contenant des cellules à transfecter, dans des conditions telles qu'il y a:
- passage du complexe macromolécule anionique/lipide cationique/acide nucléique à partir du milieu dans le cytoplasme des cellules,
- relargage de l'acide nucléique impliqué dans le susdit complexe dans le cytosol et/ou le noyau des cellules.

Comme indiqué ci-avant, les compositions selon l'invention peuvent être utilisées pour le transfert d'acides nucléiques dans les cellules *in vivo, in vitro* ou *ex vivo*. En particulier les compositions selon l'invention peuvent être utilisées pour transférer de manière très efficace des acides nucléiques dans de nombreux types cellulaires, et en particulier dans certains types cellulaires habituellement difficiles à transfecter.

Ainsi la présente invention concerne un procédé pour transférer un acide nucléique dans des cellules, ledit procédé comprenant la mise en contact desdites cellules avec une composition selon l'invention. De préférence, ledit procédé est un procédé *in vitro* ou *ex vivo*.

Les cellules peuvent être de type eucaryote, en particulier animale, végétale ou humaine.

Dans un mode de réalisation particulièrement avantageux, la composition selon la présente invention comprend en outre un élément de ciblage permettant d'orienter le transfert de l'acide nucléique, tel qu'un élément de ciblage intracellulaire (noyau, etc.) et/ou extracellulaire (ciblage de certains types cellulaires/tissus).

Parmi les éléments de ciblage utilisables dans le cadre de l'invention, on peut citer les sucres, les peptides, les oligonucléotides, les lipides ou les protéines. Avantageusement, il s'agit de sucres, de peptides ou de protéines tels que des anticorps ou fragments d'anticorps, des ligands de récepteurs cellulaires ou des fragments de ceux-ci, des récepteurs ou fragments de récepteurs, etc. En particulier, il peut s'agir de ligands de récepteurs de facteurs de croissance, de récepteurs de cytokines, de récepteurs de lectines cellulaires ou de récepteurs de protéines d'adhésion. On peut également citer le récepteur de la transferrine, des HDL et des LDL. L'élément de ciblage peut également être un sucre permettant de cibler les récepteurs asialoglycoprotéiques, ou encore un fragment Fab d'anticorps permettant de cibler le récepteur du fragment Fc des immunoglobulines.

La présente invention a également pour objet une composition pharmaceutique comprenant une composition selon la présente invention et un véhicule pharmaceutiquement acceptable. Une telle composition pharmaceutique peut être formulée pour l'administration aux mammifères, y compris l'homme. La posologie varie selon le traitement et selon l'affection en cause. Une telle composition pharmaceutique est réalisée de façon à pouvoir être administrée par la voie sous-cutanée, intramusculaire, intraveineuse, transdermique, intranasale intraoculaire, orale, sublinguale, locale, rectale, intratumorale, intrathécale, intracérébroventriculaire, intrapéritonéale, ou par instillation dans la vessie. On utilise par exemple en tant que véhicule pharmaceutiquement acceptable des suspensions aqueuses, des solutions salines isotoniques ou des solutions stériles et injectables qui contiennent des agents de dispersion et/ou des agents mouillants pharmacologiquement compatibles.

Les doses d'acide nucléique utilisées ainsi que le nombre d'administrations peuvent être adaptées en fonction de différents paramètres, et notamment en fonction du mode d'administration utilisé, de la pathologie concernée, de l'acide nucléique à administrer, ou encore de la durée du traitement recherchée.

La présente invention concerne de plus l'utilisation d'une composition vecteur comprenant :
(1) une macromolécule anionique à l'exception des acides nucléiques, et
(2) un lipide cationique tel que défini ci-dessus,
pour la préparation d'une composition destinée à la délivrance intracellulaire d'acide nucléique de taille inférieure ou égale à 200 nucléotides, ladite molécule anionique, ledit lipide cationique et ledit acide nucléique étant associés de manière non covalente.

La présente invention concerne également l'utilisation d'une composition vecteur comprenant une macromolécule anionique et un lipide cationique en tant que vecteur pharmaceutiquement acceptable d'un acide nucléique de taille inférieure ou égale à 200 nucléotides, tel qu'un ARN interférant, ladite molécule anionique, ledit lipide cationique et ledit acide nucléique étant associés de manière non covalente. La macromolécule anionique, le lipide et l'acide nucléique de taille inférieure ou égale à 200 nucléotides, sont tels que définis ci-dessus.

La présente invention concerne en outre une composition selon l'invention pour son utilisation en tant que médicament. De préférence, ledit médicament est destiné au traitement d'une pathologie associée à un dysfonctionnement d'une fonction protéique.

Dans la présente invention, on entend par « pathologie associée à un dysfonctionnement d'une fonction protéique », une pathologie liée à une ou plusieurs protéines dont la fonction est perturbée (catalyse, transport, communication, signalisation, reconnaissance, structure, ...).

Ladite pathologie peut être choisie parmi les maladies inflammatoires, telles que la polyarthrite, les rhumatismes, les cancers, les infections virales telles que l'hépatite, les infections parasitaires ou microbiennes, les maladies autoimmunes telles que le lupus érythémateux, la fibrose cardiaque ou hépatique, les maladies monogéniques liées à des mutations dominantes telles que la Chorée de Huntington, l'achondroplasie, la sclérose latérale amyotrophique ou la maladie de Lou Gehrig, les pathologies liées à un saut d'exon telles que la myopathie de Duchenne et la polymyosite, les maladies neurodégénératives telles que la maladie d'Alzheimer.

De préférence, la pathologie est choisie parmi la polyarthrite, les rhumatismes, les cancers, l'hépatite, le lupus érythémateux, la fibrose cardiaque ou hépatique, la Chorée de Huntington, l'achondroplasie, la sclérose latérale amyotrophique ou la maladie de Lou Gehrig, la myopathie de Duchenne et la polymyosite, et la maladie d'Alzheimer. De manière particulièrement préférée, la pathologie est choisie parmi la polyarthrite, l'hépatite, le lupus érythémateux, la fibrose cardiaque ou hépatique, la myopathie de Duchenne ou la polymyosite.

Ces diverses pathologies pourront être traitées à l'aide des acides nucléiques de petite taille tels que définis dans la présente invention présents dans la composition, lesquels acides nucléiques sont capables de moduler une fonction protéique.

A titre d'exemple, les fonctions protéiques pouvant être modulées à l'aide d'acides nucléiques de petite taille selon la présente invention sont celles liées à :
- TNFalpha, IL-1, IL-6, IL-18, Akt, GG2-1, ASC pour la polyarthrite rhumatoïde,
- interféron-alpha et IL-10 pour le lupus érythémateux,
- TNFalpha pour la polymyosite,
- TNF alpha pour la maladie de Crohn,
- aux gènes viraux lors d'une infection virale comme la région de Core du virus de l'hépatite B,
- aux gènes EMCV-IRE5 (« encephalomyocarditis virus-internal ribosome entry site »), NS3 et NS5B, (« non-structural protein 3 et 5B »), NA (neuraminidase) dans l'hépatite C,
- aux gènes de la caspase 8 et Fas dans l'insuffisance hépatique aiguë (ALF),
- aux gènes TGF, PDGF et TNF dans la fibrose hépatique,
- aux gènes VEGF, VEGFR1, VEGFR2 dans la dégénérescence maculaire liée à l'âge (AMD),
- aux gènes VEGF, VEGFR1, VEGFR2 dans la rétinopathie diabétique,
- aux gènes VEGF, VEGFR1, VEGFR2 dans les cancers (stratégies antiangiogénèse et antivasculaire),
- au gène FGFR3 dans l'achondroplasie,
- au gène SOD1 dans la sclérose latérale amyotrophique,
- au gène HD dans la chorée de Huntington,
- au gène de la dystrophine dans la myopathie de Duchenne (par saut d'exon),
- au gène de la β-secrétase (BACE1) dans la maladie d'Alzheimer.

Selon un dernier aspect, la présente invention se rapporte à un procédé de préparation d'une composition selon la présente invention, comprenant :
a) une étape de mise en contact de la macromolécule anionique telle que définie ci-dessus avec l'acide nucléique tel que défini ci-dessus, pour la formation d'un mélange,
b) une étape de mise en contact du lipide cationique tel que défini ci-dessus avec le mélange obtenu à l'étape a), pour la préparation de la composition.

La présente invention se rapporte également à une méthode de traitement d'une pathologie associée à un dysfonctionnement d'une fonction protéique par administration au sujet à traiter d'une composition selon l'invention.

L'invention sera mieux comprise à la lecture des exemples et des figures qui suivent.

### LEGENDE DES FIGURES

La **figure 1** **(A** et **B)** représente le pourcentage d'inhibition de l'expression de la luciférase par différentes formulations de siRNA en présence ou non d'une macromolécule anionique.
La **figure 2** représente le pourcentage d'inhibition de l'expression de la luciférase par différentes formulations de siRNA en fonction de la quantité de macromolécule anionique présente.
La **figure 3** représente le pourcentage d'inhibition de l'expression de la luciférase par différentes formulations de siRNA en fonction du degré de polymérisation de la macromolécule anionique.
La **figure 4** représente le pourcentage d'inhibition de l'expression de la luciférase par différentes formulations de siRNA en fonction du type de vecteur (micelle/liposome) et de la présence ou non de sérum.
La **figure 5** représente le pourcentage d'inhibition de l'expression de la luciférase par différentes formulations de siRNA en fonction du lipide cationique utilisé en présence et en l'absence de sérum.
La **figure 6** représente le pourcentage d'inhibition du taux de TNFalpha dans le sang chez la souris.
La **figure 7** (A et B) représente la complexation de siRNA en présence d'une macromolécule anionique et de lipides cationiques formés avec des concentrations croissantes en lipides en l'absence de PEG (7A) ou en présence de PEG (7B)
La **figure 8** (A, B et C) représente la taille des lipoplexes selon l'invention formés avec des concentrations croissantes en lipides en fonction de la quantité et du type de macromolécule anionique et du pourcentage de PEG.
La **figure 9** (A, B et C) représente la morphologie et la structure des lipoplexes selon l'invention en présence ou en l'absence de PEG
La **figure 10** représente l'efficacité d'inhibition de la luciférase par des lipoplexes selon l'invention en fonction du ratio de charges, du type et de la quantité de macromolécules anioniques.
La **figure 11** (A et B) représente l'efficacité de transfection de lipoplexes selon l'invention, par l'étude de l'expression d'un gène endogène (RIP ou TNFR1).
La **figure 12** (A et B) représente la stabilité physique de lipoplexes selon l'invention en présence ou en l'absence de PEG
La **figure 13** représente la cytotoxicité des lipoplexes selon l'invention.
La **figure 14** (A, B et C) représente la biodistribution *in vivo* de lipoplexes selon l'invention

### EXEMPLES

### Exemple 1 : Matériels et méthodes

Le composé 2- {3-[Bis-(3-amino-propyl)-amino]-propylamino}-N-ditetradecyl carbamoyl methyl-acetamide (RPR209120) est synthétisé au laboratoire selon le protocole décrit dans le brevet US 6,171,612.

Le composé RPR120535 (2-{3-[3-(3-Amino-propylamino)-propylamino]-propylamino}-N,N-dioctadecyl-acetamide), est synthétisé au laboratoire selon le protocole décrit dans l'article Byk et al. (1998, J. Med. Chem., 41, 224-235).

Le RPR209120 et la DOSPA (GIBCO BRL) sont des lipopolyamines, préparées en liposome avec la DOPE. Le DMRIE (GIBCO BRL) est un ammonium quaternaire (1,2-dimyristyloxypropyl-3-dimethyl-hydroxy ethyl ammonium bromide), préparé en liposome avec du cholestérol (DMRIE-C).

La dioleyl phosphatidyl ethanolamine (DOPE) a été achetée chez AVANTI POLAR LIPIDS.

### Préparation des liposomes cationiques 209120/DOPE

Le lipide RPR209120 (2-{3-[Bis-(3-amino-propyl)-amino]-propylamino}-N-ditetradecyl carbamoyl methyl-acetamide), synthétisé au laboratoire selon le protocole décrit dans le brevet US 6,171,612 et la DOPE (dioleyl phosphatidyl éthanolamine) achetée chez AVANTI POLAR LIPIDS, sont dissous dans du chloroforme en quantité équimolaire dans un petit ballon. Le solvant organique est évaporé sous vide à 20°C en utilisant un évaporateur rotatif de type Heidolph de façon à former un film lipidique au fond du ballon. Le film est séché pendant 2h puis hydraté avec de l'eau pour préparation injectable (ppi) pendant 4h à 20°C puis une nuit à 4°C pour former des vésicules multilamellaires de grandes tailles. La suspension est soumise à une sonication (115V, 80W, 50-60Hz, appareil LABORATORY SUPPLIES) jusqu'à obtenir une suspension homogène de liposomes présentant un diamètre de 80-100 nm.

Des liposomes RPR209 120 DOPE (1 :1) +/- lipide -PEG2000 à 1, 3 et 5% (poids/poids de lipide total) sont également préparés. Une quantité adéquate de DSPE-PEG2000, produit acheté chez Avanti Polar, est ajoutée au lipide cationique et DOPE avant dissolution dans du chloroforme dans le ballon.

### Préparation des acides nucléiques

Les siRNAs synthétiques sont achetés chez QIAGEN. Ils sont hybridés selon les recommandations du fabricant avant toute utilisation. Les siRNAs sont en solution dans le tampon fourni par le fabricant à une concentration de 100µM.

Séquences du brin sens :
Luc 5'CUUACGCUGAGUACUUCGA3' (ciblé vers la luciférase) : SEQ ID NO : 1.
CTLE 5'UUCUCCGAACGUGUCACGU3' (aucune cible) : SEQ ID NO : 2.
TNFalpha 5'GACAACCAACUAGUGGUGCTT3' (ciblé vers le TNFalpha de souris) : SEQ ID NO : 3.

Les oligonucléotides synthétiques sont achetés chez EUROGENTEC. Ils sont fournis en solution à 100µM. Leur séquence est telle qu'ils peuvent s'hybrider pour former une macromolécule de grande taille par répétition de leur motif (oligo1 : 5'ATGTACTTAGCTAGT3' (SEQ ID NO : 4), oligo2 : 5'TACATACTAGCTAAG3' (SEQ ID NO : 5)).

### Les macromolécules anioniques

L'acide poly-L-glutamique (sel de sodium) ou APG est acheté chez SIGMA ALDRICH: (1) APG-60k, degré de polymérisation 371, poids moléculaire moyen 56000, (2) APG-4k, degré de polymérisation 28, poids moléculaire moyen 4300. L'acide alginique (sel de sodium) est acheté chez SIGMA ALDRICH.

Le sulfate de dextran (sel de sodium) est acheté chez SIGMA ALDRICH, SD.

Le carboxymethyl cellulose (sel de sodium) est acheté chez SIGMA ALDRICH, CMC. Les macromolécules anioniques sont préparées en solution dans l'eau à une concentration de 5 mg/ml.

### Préparation des lipoplexes (complexes acide nucléique / lipide cationique et complexes macromolécule anionique / acide nucléique / lipide cationique)

Les lipoplexes sont préparés en mélangeant volume à volume une suspension de liposomes cationiques ou de micelles cationiques (à la concentration appropriée dans du NaCl 150mM) à une solution d'acides nucléiques (ADN plasmidique, siRNA) préparée également dans du NaCl 150mM. Le mélange se fait sous forte agitation, 30 minutes à température ambiante. Lorsqu'une macromolécule anionique est ajoutée à la formulation, elle est mélangée avec les siRNAs dans du NaCl 150 mM avant le mélange volume à volume avec les liposomes cationiques ou les micelles cationiques. Les lipoplexes préparés avec la LipofectAMINE ou le DMRIE-C sont préparés comme décrit ci-dessus mais le NaCl 150mM est remplacé par de l'OptiMEM (INVITROGEN). Le ratio de charge correspond au nombre de charges positives apportées par le ou les lipides divisé par le nombre de charges négatives apportées par les siRNA et la macromolécule anionique.

### Détermination de la taille et du potentiel Zêta des complexes

La taille et le potentiel Zêta des liposomes et des particules ont été déterminés à l'aide du Zetasizer 3000 (MALVERN). Les liposomes et lipoplexes sont préparés dans du NaCl 150mM à une concentration finale en siRNA (pour les lipoplexes) de 700nM.

### Lignée cellulaire

Une lignée de mélanome de souris (B16-F0, ATCC, CRL-6322) a été modifiée pour exprimer de manière constitutive un gène rapporteur, la luciférase de luciole (Luc+, PROMEGA) sous le contrôle du promoteur du virus SV40. Après électroporation des cellules en présence du plasmide portant le gène luc+ et un gène de résistance à la généticine, les cellules sont sélectionnées pour leur résistance à la généticine, puis une lignée cellulaire clonale est obtenue par dilution limite des cellules résistantes et exprimant la luciférase. Les cellules sont maintenues en culture dans du DMEM contenant du Glutamax (FISHER BIOBLOCK), 10% sérum de veau foetal, de la streptomycine (100 µg/ml) et de la pénicilline (100 U/ml) à 37°C en présence de 5% de CO₂.

### Transfection

La veille, les cellules B16-Luc sont ensemencées en plaques 24-puits à la densité de 40 000 cellules par puits. Le jour même, les cellules sont rincées avec du DMEM puis mises en incubation dans le milieu de transfection. Le milieu de transfection contient les lipoplexes préparés comme indiqué ci-dessus et dilués au dixième dans du milieu de culture des cellules lorsque la transfection est réalisée en présence de sérum ou dans du DMEM lorsque la transfection est réalisée en absence de sérum. Dans ce dernier cas, 10% de sérum est ajouté au milieu de transfection 4h après le début de la transfection. Les cellules sont mises en incubation à 37°C en présence de 5% de CO₂. 24h après le début de la transfection, le milieu de transfection est remplacé par du milieu de culture frais puis les cellules sont mises en incubation pour une période supplémentaire de 24h.

### Dosages de l'activité luciférase et des protéines

A la fin de la transfection, les cellules sont rincées deux fois dans du PBS puis lysées avec 200µl de Cell Culture Lysis Reagent (PROMEGA). Après récupération du lysat et centrifugation pour éliminer les débris cellulaires, l'activité luciférase est mesurée sur le lysat après ajout du substrat (Luciferase Assay Substrate, PROMEGA) et lecture de la lumière émise à l'aide d'un lecteur de microplaque équipé pour la luminescence (WALLAC VICTOR, PERKIN ELMER). L'activité obtenue, exprimée en cps (coups par seconde) est normalisée par la concentration en protéines présentes dans le lysat et déterminée par un test au BCA (INTERCHIM).

### Exemple 2 : Méthodes d'évaluation des macromolécules anioniques

Les tests cellulaires sont réalisés sur une lignée de cellules de souris qui expriment de façon continue une protéine « rapportrice », c'est-à-dire dont l'activité est facile à mesurer avec précision et de manière reproductible. Il s'agit de la luciférase qui émet de la lumière, facilement mesurable, lorsqu'on lui fournit sa molécule substrat, la luciférine. Les cellules exprimant la luciférase sont mises en présence de différentes préparations contenant un siRNA capable d'inhiber spécifiquement l'expression de cette luciférase associée à un vecteur. La lumière émise par ces cellules 48h après le traitement est mesurée et comparée avec des cellules n'ayant subi aucun traitement. Les compositions comprenant le liposome cationique, l'acide nucléique et la macromolécule anionique sont préparées pour des concentrations allant de 3 à 8 nmoles de lipide cationique par µg d'acide nucléique et de macromolécule anionique, la concentration finale utilisée en siRNA est de 20nM. Des compositions préparées dans les mêmes conditions mais contenant un siRNA contrôle (sans activité d'inhibition) sont testées en parallèle de chaque expérience afin d'observer l'activité non spécifique. Dans la majorité des cas présentés ci-dessous, ces compositions ne conduisent à aucune inhibition de l'activité luciférase.

### Exemple 3 : Identification de la macromolécule anionique

### Protocole

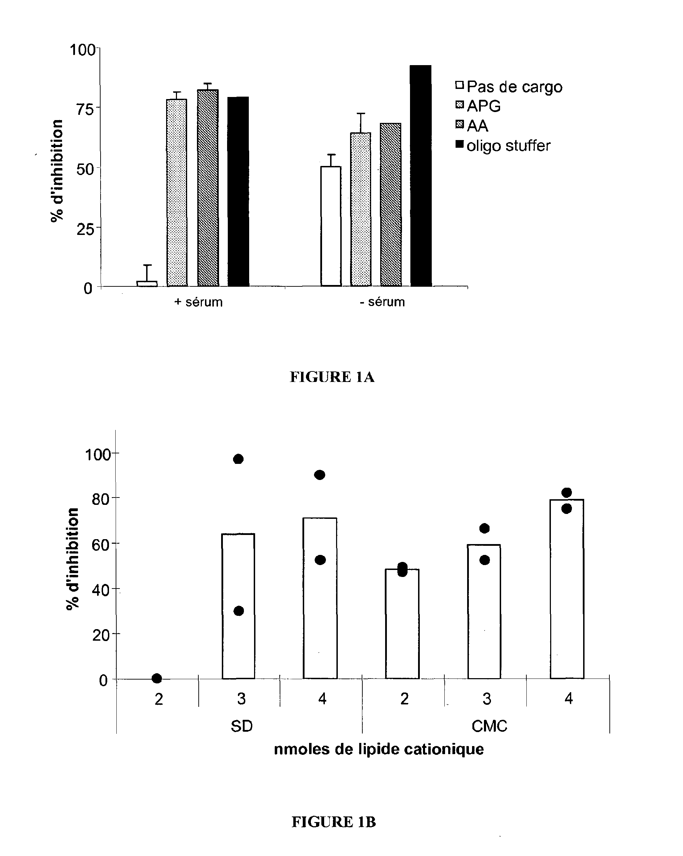

0,3 µg de siRNA spécifique de la luciférase sont mélangés avec 0,3 µg de différentes macromolécules anioniques (APG = acide polyglutamique, AA = acide alginique, SD = sulfate de dextran, CMC = carboxymethyl cellulose) puis associés au liposome cationique RPR209120/DOPE (figure 1A : 4 nmoles de lipide cationique/µg de mélange (siRNA+ macromolécule anionique) ; figure 1B : 2, 3, 4 nmoles de lipide cationique/µg de mélange (siRNA+ macromolécule anionique), avant d'être placés au contact des cellules pendant 24h. L'activité luciférase est mesurée 48h après le traitement. Les résultats sont exprimés en pourcentage d'inhibition par rapport à des cellules n'ayant reçu aucun traitement (figure 1A) ou ayant reçu du siRNA contrôle préparé selon la même formulation (figure 1B).

Concernant les résultats représentés sur la figure 1A, le traitement est réalisé soit dans le milieu naturel complet des cellules (+ sérum), soit en enlevant momentanément le sérum, source de protéines essentielles pour la croissance des cellules (- sérum). La seconde condition est souvent utilisée car elle permet d'éliminer l'interférence des protéines du sérum avec les préparations mais elle est très éloignée des conditions dans un organisme. La première condition est donc plus proche des conditions attendues dans un organisme.

Concernant les résultats représentés sur la figure 1B, le traitement est réalisé dans le milieu naturel complet des cellules (+ sérum).

### Résultats

Les résultats sont présentés sur la figure 1 (A et B).

On constate que les formulations avec une macromolécule anionique sont aussi efficaces en présence de sérum qu'en absence de sérum alors que la formulation sans macromolécule anionique n'est efficace qu'en absence de sérum, ce qui justifie d'ajouter une macromolécule anionique à la formulation pour obtenir une formulation susceptible d'être efficace dans l'organisme. Les figures 1A et 1B montrent que l'ensemble des polymères anioniques testés (à savoir, l'acide polyglutamique, l'acide alginique, le sulfate de dextran et le carboxymethyl cellulose) est efficace pour permettre l'inhibition de la luciférase. De plus, sur la figure 1A, on observe que l'acide polyglutamique et l'acide alginique permettent une augmentation de l'extinction de gène équivalente entre eux. Cette extinction est spécifique car aucune extinction n'est obtenue quand le siRNA ciblé vers la luciférase est remplacé par un siRNA contrôle, n'ayant aucune cible.

### Exemple 4 : Effet de la quantité de macromolécule anionique ajoutée

### Protocole

0,3 µg de siRNA spécifique de la luciférase sont mélangés avec différentes quantités d'acide polyglutamique (APG) c'est-à-dire 0,15, 0,3, 0,6 et 1,2 µg d'APG puis associés au liposome cationique RPR209120/DOPE (4 nmoles de lipide cationique/µg de mélange siRNA+ macromolécule anionique), avant d'être placés au contact des cellules pendant 24h. L'activité luciférase est mesurée 48h après le traitement. Les résultats sont exprimés en pourcentage d'inhibition par rapport à des cellules n'ayant reçu aucun traitement.

### Résultats

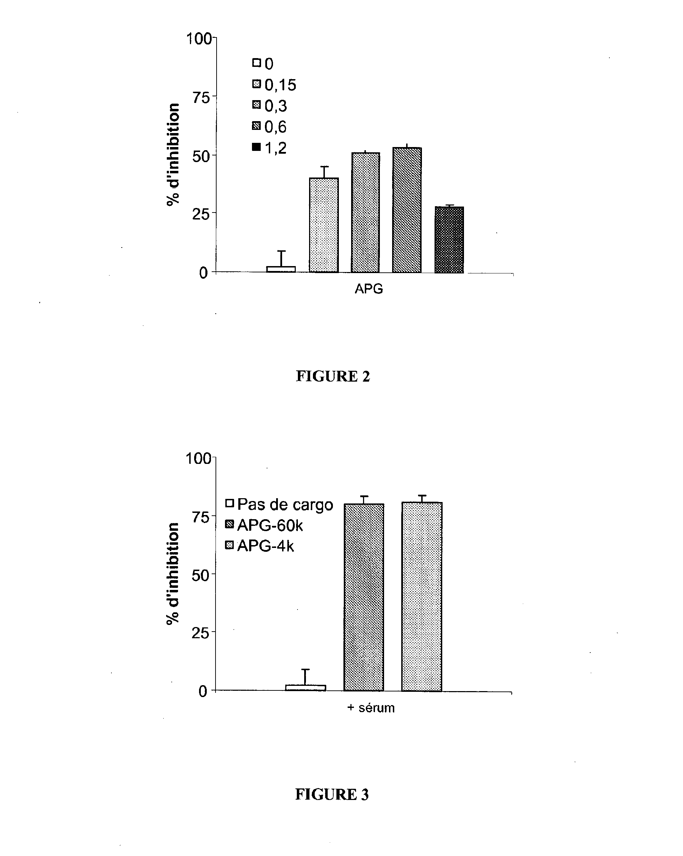

Les résultats, présentés sur la figure 2, montrent l'effet de la quantité de macromolécule ajoutée sur l'inhibition. On obtient sensiblement la même efficacité avec un rapport poids/poids siRNA/macromolécule de 1/0,5 à 1/2. Pour un rapport de 1/4, la diminution de l'inhibition peut être expliquée par la toxicité des formulations sur les cellules, qui comprennent 4 fois plus de lipides que dans le cas du rapport 1/1.

### Exempte 5 : Effet de la taille de la macromolécule anionique

0,3 µg de siRNA spécifique de la luciférase sont mélangés avec 0,3 µg de différents macromolécules anioniques (ADN, APG-60k (degré polymérisation 371, poids moléculaire moyen 56000), APG-4k (degré polymérisation 28, poids moléculaire moyen 4300) puis associés au liposome cationique RPR209120/DOPE (4 nmoles de lipide cationique/µg de mélange siRNA+ macromolécule anionique), avant d'être placés au contact des cellules pendant 24h. L'activité luciférase est mesurée 48h après le traitement. Les résultats sont exprimés en pourcentage d'inhibition par rapport à des cellules n'ayant reçu aucun traitement.

### Résultats

Les résultats, présentés sur la figure 3, montrent que le pourcentage d'inhibition est indépendant du degré de polymérisation de la macromolécule anionique.

### Exemple 6 : Effet du mode de préparation du vecteur lipidique

### Protocole

0,3 µg de siRNA spécifique de la luciférase sont mélangés avec 0,3 µg de différentes macromolécules anioniques (ADN, APG, AA) puis associés au lipide cationique RPR120535 préparé en liposome (par association avec un lipide neutre comme la DOPE) ou en micelles (4 nmoles de lipide cationique/µg de mélange siRNA+ macromolécule anionique), avant d'être placés au contact des cellules pendant 24h. L'activité luciférase est mesurée 48h après le traitement. Les résultats sont exprimés en pourcentage d'inhibition par rapport à des cellules n'ayant reçu aucun traitement.

### Résultats

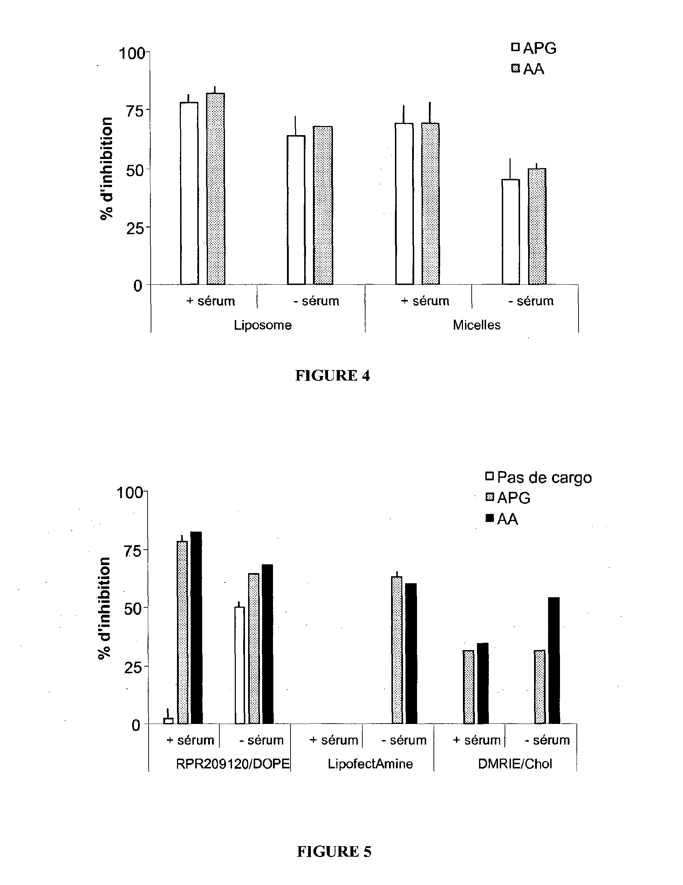

Les résultats, présentés sur la figure 4, montrent que l'ajout de macromolécule anionique dans la formulation permet d'obtenir une bonne efficacité d'inhibition de la luciférase, que le lipide cationique soit préparé sous forme de liposome ou sous forme de micelles.

### Exemple 7 : Effet du types de lipide cationique

### Protocole

0,3 µg de siRNA spécifique de la luciférase sont mélangés avec 0,3 µg de différentes macromolécules anioniques (ADN, APG, AA) puis associés à différents lipides cationiques aux conditions optimales de transfection pour chacun (RPR209120/DOPE, 4 nmoles / µg de mélange siRNA + macromolécule anionique; LipofectAmine et DMRIE-C, 5µl / µg de mélange (siRNA+ macromolécule anionique) avant d'être placés au contact des cellules pendant 24h. L'activité luciférase est mesurée 48h après le traitement. Les résultats sont exprimés en pourcentage d'inhibition par rapport à des cellules n'ayant reçu aucun traitement.

### Résultats

Les résultats, présentés sur la figure 5, montrent que l'addition d'une macromolécule anionique pour augmenter l'efficacité d'inhibition de l'expression d'un gène est applicable à d'autres lipides cationiques que le RPR209120. La Lipofectamine n'est pas efficace en présence de sérum, phénomène déjà démontré dans la littérature et bien connu. Le DMRIE est moins efficace que le RPR209120.

### Exemple 8 : Effet de la macromolécule anionique sur un modèle animal

### Protocole

Des souris Swiss femelles âgées de 5 semaines sont traitées par une injection intrapéritonéale de Lipopolysaccharides (LPS d'E coli, Sigma Aldrich) en NaCl 150mM selon une dose de 4mg/kg. Un prélèvement du sang dans le sinus rétro-orbital est réalisé 1h30 plus tard. Le dosage du TNFalpha présent dans le sang est réalisé sur 100µl de sérum à l'aide du kit de dosage DuoSet Mouse TNFalpha (R&D Systems). 24h avant le traitement par les LPS, les souris reçoivent en intrapéritonéale 5µg de siRNAs formulés en lipoplexes avec le liposome cationique RPR209120/DOPE (6 nmoles de lipide cationique / µg de (siRNA+ macromolécule anionique)). Il est soit formulé seul, soit après pré-association avec l'acide polyglutamique (PGLA) ou l'acide alginique (AA) avant l'association avec le liposome cationique. Les résultats sont exprimés en pourcentage d'inhibition par rapport à des souris traitées avec la même formulation contenant un siRNA contrôle.

### Résultats

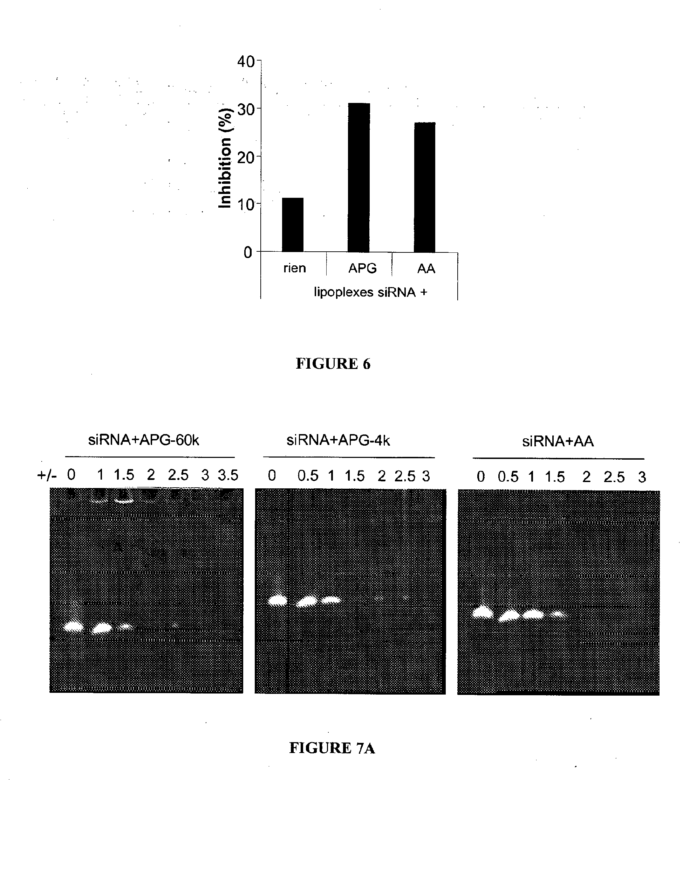

La figure 6 montre les taux d'inhibition obtenus.

Les résultats montrent qu'un prétraitement des souris par des siRNA dirigés contre le TNFalpha permet de diminuer ce taux de TNFalpha dans le sang. La diminution observée est améliorée par la présence de la macromolécule anionique dans la formulation.

### Exemple 9 : Etude de la complexation de siRNA en présence d'une macromolécule anionique et de doses croissantes de lipides cationiques en l'absence ou en présence de PEG

### Protocole

Des liposomes RPR209120:DOPE (1:1) en présence ou en l'absence de PEG₂₀₀₀ à 1, 3 et 5% (mole/mole de lipide total).sont préparés par sonication.

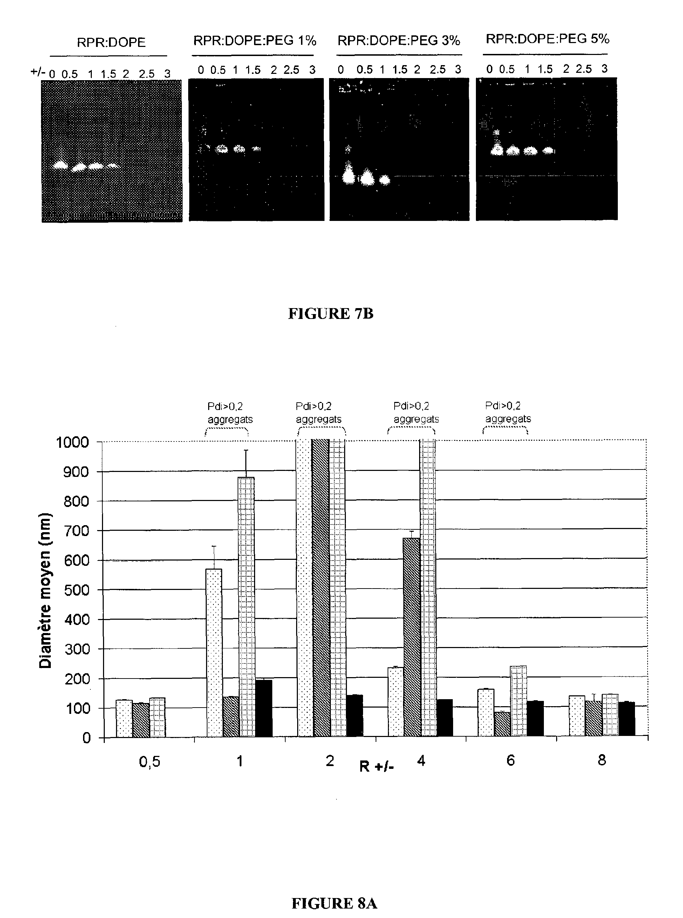

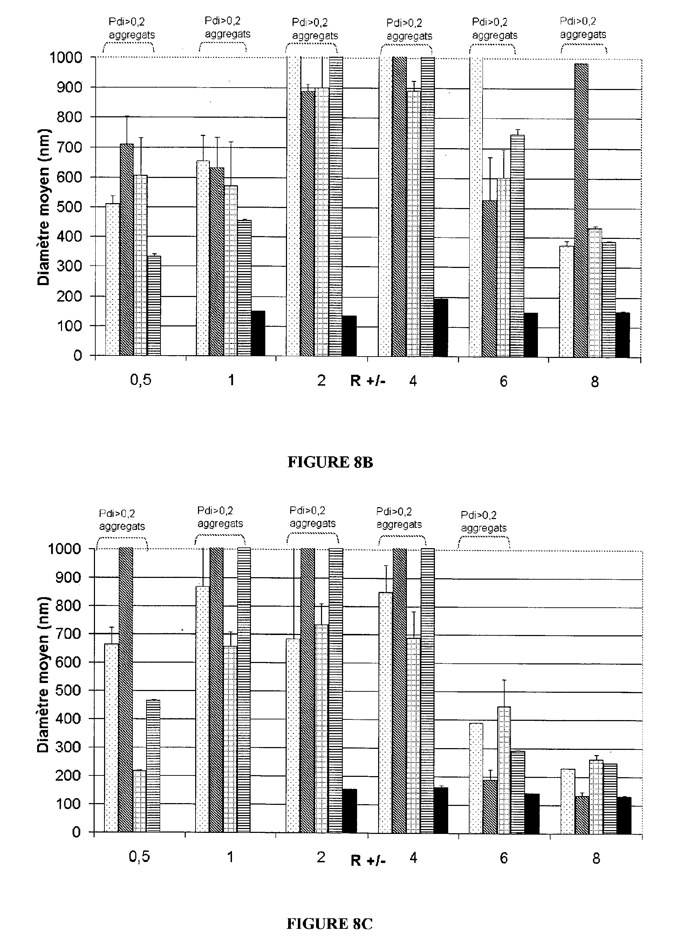

Des lipoplexes formés avec des concentrations croissantes en lipides cationiques sont préparés (ratio de charges de 0.5 à 3) et analysés sur un gel de polyacrylamide en présence d'urée. A la fin de la migration, les gels sont placés dans un bain de SybrGreen pour colorer l'ARN. Les résultats sont présentés sur les figures 7 et 8.

Ainsi, la Figure 7 montre les profils de complexation des siRNA par les formulations RPR:DOPE contenant les différents macromolécules anioniques dans du NaCl 150mM en fonction du ratio de charges. Le ratio siRNA/polymère est de 1/1 w/w.

La figure 8 montre le profil de complexation des siRNA par des formulations RPR:DOPE:PEG 0-5% contenant de l'acide alginique dans du NaCl 150mM en fonction du ratio de charges. Le ratio siRNA/polymère est de 1/1 w/w.

### Résultats

Les résultats présentés sur la figure 7 montrent que l'ajout d'un polymère anionique quel qu'il soit ne modifie pas les capacités d'interaction des siRNA avec les lipides cationiques.

Les résultats présentés sur la figure 8 montrent que l'ajout de PEG ne modifie pas les capacités d'interaction des siRNA avec les lipides cationiques. La même observation est faite quelque soit le polymère anionique utilisé. Les siRNA sont totalement complexés à un ratio de charges supérieur à 2 pour toutes les lipolexes testés.

### Exemple 10 : Etude de la taille des lipoplexes en fonction de doses croissantes de lipides cationiques, en l'absence ou en présence de PEG, en fonction du ratio siRNA/macromolécule anionique, avec différentes macromolécules anioniques

### Protocole

La taille des lipoplexes (siRNA/macromolécule anionique formulées avec du RPR:DOPE:PEG 0-5%) a été mesurée (par diffusion quasi élastique de la lumière dans un appareil Zeta Malvern) dans du NaCl 150mM en fonction du ratio de charges, de la quantité de macromolécule anionique (figure 8A : l'acide polyglutamique APG-60k ; figure 8 B : l'acide polyglutamique APG-4k ; figure 8 C : l'acide alginique) et du pourcentage de PEG pour chaque macromolécule anionique. Le ratio siRNA/polymère (w/w) a été modifié de 1/0.5 à 1/2.

### Résultats

La figure 8 montre que l'ajout de PEG favorise la formation de plus petites particules et ce quelque soit la macromolécule anionique utilisée dans les lipolexes selon l'invention. Les lipoplexes ont une taille d'environ 150nm avec 5% de PEG, quelque soit le ratio de charges. L'ajout de seulement 1% de PEG ne permet pas de stabiliser suffisamment les particules, elles s'agrègent, de la même manière qu'avec les formulations sans PEG.

Les différentes macromolécules anioniques testées montrent des profils de stabilité colloïdale différents. On retrouve dans tous les cas une diminution de la taille des lipoplexes lorsque le ratio de charges augmente. A des ratios de charges élevés, de l'ordre de 6-8, les lipoplexes sont bien plus petits avec l'acide polyglutamique APG-60k et acide alginique.

### Exemple 11 : Etude de la morphologie et de la structure des lipoplexes en l'absence ou en présence de PEG avec différentes macromolécules anioniques

### Protocole

La morphologie des lipoplexes (siRNA/macromolécules anioniques formulées avec du RPR:DOPE:PEG 0 ou 5% dans du NaCl 150mM) préparés avec un ratio de charges égal à 6 avec différentes macromolécules anioniques a été analysée par microscopie électronique à transmission (MET).

### Résultats

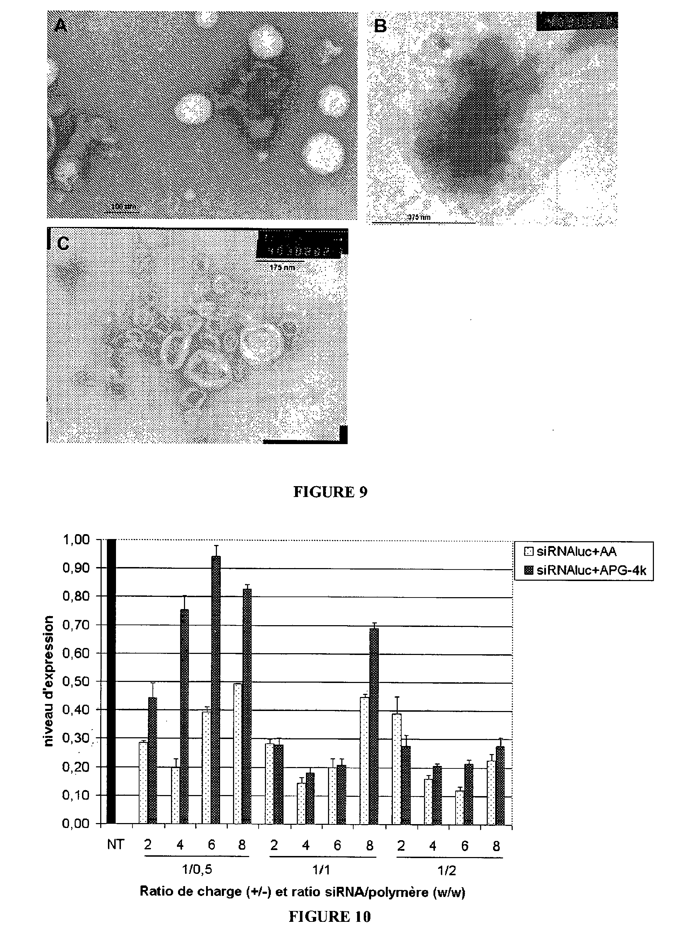

Les clichés sont présentés sur la figure 9 (Figure 9A acide alginique en l'absence de PEG ; Figure 9B : acide polyglutamique (APG-4k) en l'absence de PEG ; Figure 9C acide polyglutamique (APG-4k) en présence de 5% de PEG).

En absence de PEG, la plupart des lipoplexes ont une structure en feuillets et semble précipités en gros amas.

En présence de PEG 5%, la plupart des lipoplexes ont une morphologie sphérique et semblent accolés les uns aux autres. La population est hétérogène, avec des tailles allant de 50 à 200 nm de diamètre en moyenne.

### Exemple 12 : Etude de l'efficacité de transfection des lipoplexes en l'absence et en présence de PEG avec différentes macromolécules anioniques

### Protocole

Des études de transfection ont été réalisées sur des cellules de mélanome B16 exprimant de manière constitutive la Luciférase (promoteur SV40). Des transfections avec différents lipoplexes (siRNA/macromolécule anionique formulées avec du RPR:DOPE dans du NaCl 150mM) ont été réalisées sur 48h, en faisant varier le ratio de charges, la quantité de macromolécules anioniques, la quantité de siRNA et l'ajout de PEG aux lipoplexes. Les expériences sont effectuées en triple, avec des siRNA dirigés contre la luciférase et des siRNA contrôle non spécifique. Les résultats en coups par seconde (cps) normalisés par la concentration en protéines totales sont exprimés par rapport aux cellules transfectées dans les mêmes conditions avec des siRNA contrôle. La quantité de protéines totales est un indicateur de la prolifération des cellules. Les expériences de transfection ont été réalisées avec 20nM de siRNA.

### Résultats

Les résultats représentés sur la figure 10 montrent que l'ajout d'une plus grande proportion de macromolécules anioniques améliore l'efficacité de transfection quelque soit le ratio de charges.

Des expériences de transfection ont également été entreprises avec les lipoplexes en présence de PEG. Une forte diminution de l'efficacité de transfection est alors observée lorsque la quantité de PEG augmente.

### Exemple 13 : Etude de l'efficacité de transfection de lipoplexes selon l'invention par l'étude de l'expression d'un gène endogène (RIP ou TNFR1)

### Protocole

Des études de transfection ont été réalisées sur des cellules de mélanome B16 exprimant de manière constitutive la Luciférase (promoteur SV40). Des transfections avec différents lipoplexes ont été réalisées sur 48h, en faisant varier le ratio de charges, la quantité de macromolécule anionique et la quantité de siRNA. Les expériences sont effectuées en triple, avec des siRNA dirigés contre un gène endogène (RIP (TNF Receptor Interacting serine threonine kinase 1) ou TNFR1 (Tumor necrosis factor receptor 1) et des siRNA contrôles non spécifiques. Après 48h, les cellules sont récoltées, les ARN totaux sont extraits au phénol/chloroforme, puis rétro-transcrits en ADNc à l'aide d'amorces aléatoires. Les ADNc sont ensuite analysés par PCR quantitative à l'aide d'amorces dirigées contre le gène d'intérêt. L'accumulation des produits PCR est suivie par la fluorescence du SybrGreen.

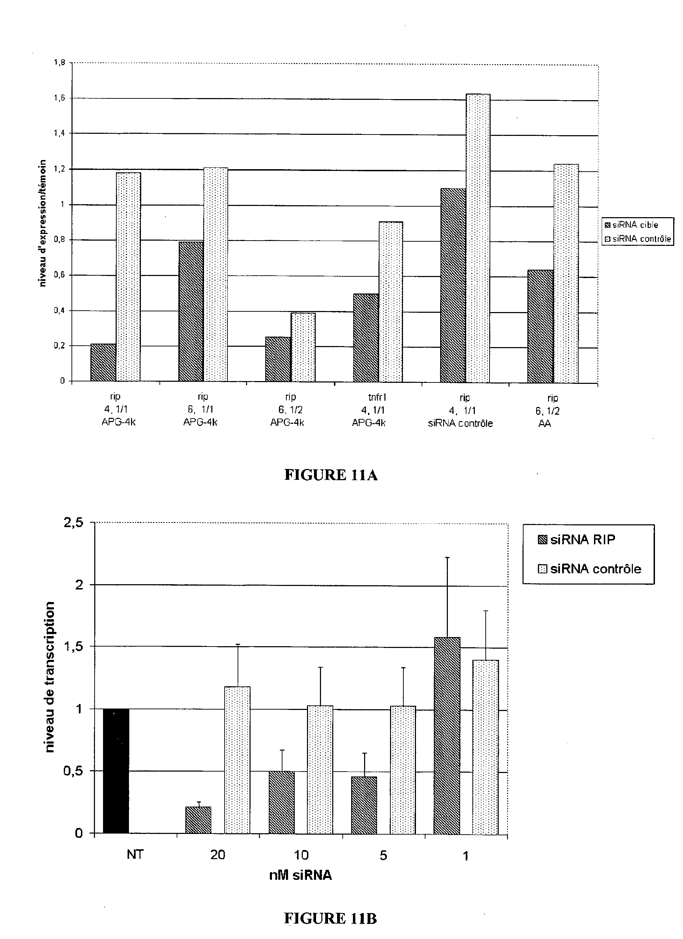

La Figure 11 A représente l'efficacité d'inhibition de RIP ou TNFR1 par les lipoplexes (siRNA/macromolécule anionique formulées avec du RPR:DOPE dans du NaCl 150mM) en fonction du ratio de charges, de la quantité de macromolécule anionique et du type de macromolécule anionique (acide polyglutamique APG-4k ou l'acide alginique) Les transfections sont réalisées avec 20nM de siRNA. La figure 11 B représente l'efficacité d'inhibition de RIP par les lipoplexes siRNA/APG-4k formulées avec du RPR:DOPE dans du NaCl 150mM à un ratio de charges de 4, 1/1 w/w en diminuant la quantité de siRNA et en la remplaçant par de la macromolécule anionique. Le niveau des cellules non transfectées est arbitrairement fixé à 1.

### Résultats

Les résultats présentés sur la figure 11A montrent une inhibition de 80% du gène RIP par rapport aux cellules non transfectées. La même expérience réalisée sur le gène TNFR1 montre seulement une inhibition de 50% montrant ainsi que les efficacités obtenues varient selon le siRNA et le gène choisi. L'ajout de macromolécule anionique à un siRNA avant formulation en lipoplexes avec un lipide cationique, que se soit de l'acide alginique ou de l'acide polyglutamique (APG-4k) permet une meilleure efficacité par rapport aux formulations sans macromolécules anioniques (avec ajout de siRNA contrôle pour conserver la même quantité de lipide).

Les résultats présentés sur la figure 11B ont été réalisés en diminuant la quantité de siRNA et en remplaçant les siRNA enlevés par de la macromolécule anionique donc en gardant toujours la même concentration en lipoplexes. De cette manière une bonne efficacité de transfection est maintenue, même avec des concentrations en siRNA allant de 20nM à 5nM.

### Exemple 14 : Etude de la stabilité de lipoplexes selon l'invention en présence ou en l'absence de PEG dans du sérum à 37°C.

### Protocole

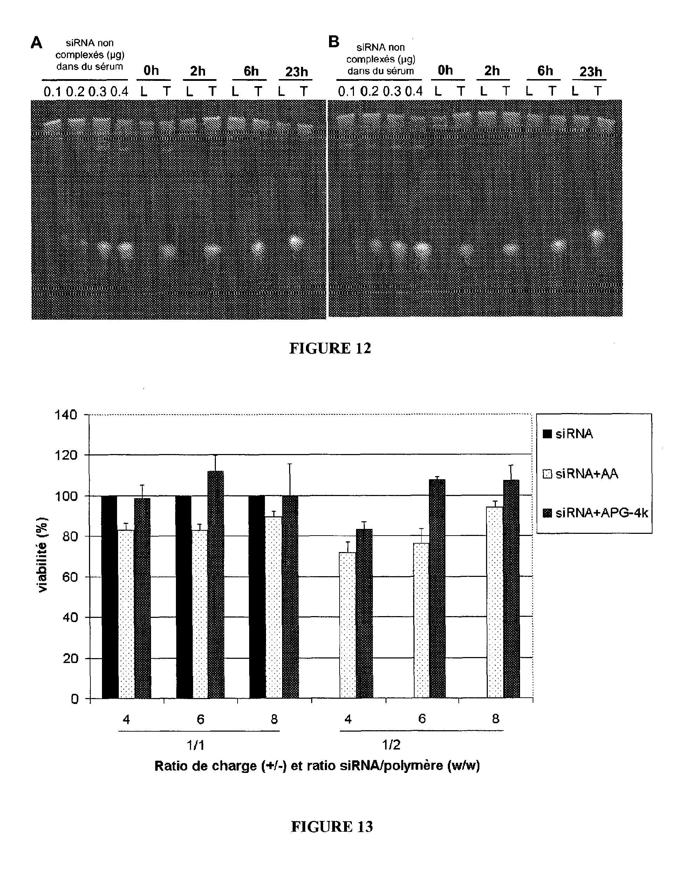

La stabilité physique des lipoplexes dans le sérum a été suivie par électrophorèse sur gel de polyacrylamide 6%-Urée et détection des siRNA au SybrGreenII. Les lipoplexes sont mis en incubation dans du sérum de veau foetal 50% et l'échantillon est ensuite dissocié par 1% Triton X-100 qui va solubiliser les lipides, libérer les siRNA et va permettre ainsi de les quantifier. La stabilité des lipoplexes dans du sérum 50% à 37°C est mesurée aux temps 0h, 2h, 6h et 23h. Les résultats sont présentés sur la figure 12.

La figure 12 représente ainsi la stabilité des lipolexes siRNA/PGLA1818 formulées avec du RPR:DOPE:PEG 0 ou 5% dans du NaCl 150mM à un ratio de charges de 6, dans du sérum 50% à 37°C. en l'absence de PEG (figure 12A), ou en présence de PEG 5% (figure 12B). Sur la figure 12, « L » correspond aux lipoplexes et « T » aux lipoplexes dissociés par le Triton..

### Résultats

Les résultats représentés sur la figure 12 montrent qu'il n'existe aucun siRNA libre en solution avant dissociation au Triton. Deux hypothèses sont envisageables : soit les lipoplexes sont stables physiquement dans le sérum et donc non dissociés, soit qu'ils se dissocient mais que les siRNA libérés sont dégradés par les nucléases du sérum. La dissociation par le Triton permet de quantifier les siRNA restés compactés par les lipides.

Nous pouvons considérer que les lipoplexes avec ou sans PEG avec de l'acide polyglutamique sont stables pendant 23h dans 50% de sérum. Les lipoplexes avec ou sans PEG avec de l'acide alginique n'ont été examinés que jusqu'à 6h d'incubation. Ils étaient stables de la même manière.

De plus l'addition d'une macromolécule anionique comme l'acide alginique ou l'acide polyglutamique n'entraine pas de déstabilisation des lipoplexes dans le sérum.

### Exemple 15 : Etude de la cytotoxicité de lipoplexes selon l'invention

### Protocole

La cytotoxicité des lipolexes selon l'invention est étudiée avec le test MTT (sel de tétrazolonium). L'anneau de tétrazolium qu'il contient est réduit, par la succinate déshydrogénase mitochondriale des cellules vivantes actives, en formazan. La couleur du milieu passe alors du jaune au bleu-violacé et la lecture est réalisée au spectrophotomètre à 562nm. L'intensité de cette coloration est proportionnelle au nombre de cellules vivantes présentes lors du test mais aussi à leur activité métabolique. Les résultats sont représentés sur la figure 13. Ainsi, la figure 13 représente la viabilité des cellules transfectées par les particules siRNA/macromolécules anioniques formulées avec du RPR:DOPE dans du NaCl 150mM en fonction du ratio de charges et de la quantité de macromolécules anioniques. Le pourcentage de viabilité est exprimé par rapport aux cellules non transfectées. La quantité de siRNA est de 20nM. La quantité de protéines totales déterminée par dosage au BCA est également représentée.

La toxicité des lipolexes a été observée après 48h avec l'acide alginique ou l'acide polyglutamique APG-4k à un ratio de charges de 4, 6 ou 8, et avec un ratio siRNA/polymère de 1/1 ou 1/2 w/w (poids/poids). Les pourcentages de viabilité obtenus ont été comparés à ceux obtenus pour des cellules transfectées avec siRNA/RPR:DOPE (la macromolécule anionique a été remplacée par du siRNA pour conserver la même quantité de lipide) et pour des cellules cultivées avec DMSO (contrôle positif de toxicité).

### Résultats

Les résultats représentés sur la figure 13 montrent que globalement l'ajout de macromolécules anioniques à un siRNA avant formulation en lipolexes avec un lipide cationique n'entraîne pas de toxicité supplémentaire ou seulement très légère avec l'acide alginique. La toxicité diminue lorsque le ratio de charges augmente.

### Exemple 16 : Etude de la biodistribution de lipoplexes selon l'invention

### Protocole

Le modèle *in vivo* utilisé est un modèle de métastases de mélanome B16 dans les poumons de souris C57/BL6.

Le protocole utilisé est le suivant :
A t=0, injection en intraveineuse de 2x10⁵ cellules B16-F0 chez 20 souris.
A t=15j, trois groupes sont obtenus :
   groupe A : 10 souris sans tumeurs (récolte des organes à 2h) groupe B : 10 souris avec tumeurs B16 (récolte des organes à 2h)
   groupe C : 10 souris avec tumeur B16 (récolte des organes à 24h)

Pour chacun des groupes :
- une injection en intraveineuse (200µL) est effectuée chez 4 souris avec 10µg de siRNA contrôle formulé à un ratio de charges égal à 8 avec du RPR:DOPE:PE-Rhodamine (appelé « lipo fluo »), et
- une injection en intraveineuse. (200µL) est effectuée chez 4 souris avec 10µg de siRNA-Rhodamine formulé à un ratio de charges égal à 8 avec du RPR:DOPE (appelé « sirna fluo »)
- les deux souris restantes servent de témoin.

Les souris sont sacrifiées 2h ou 24h après l'injection des lipoplexes et les organes sont observés au Macrofluo Leica (loupe binoculaire équipée en fluorescence), puis :
- les organes des souris ayant reçu des liposomes fluo sont conservés à -20°C
- les organes des souris ayant reçu des siRNA fluo sont conservés dans du RNAlater® à 4°C.

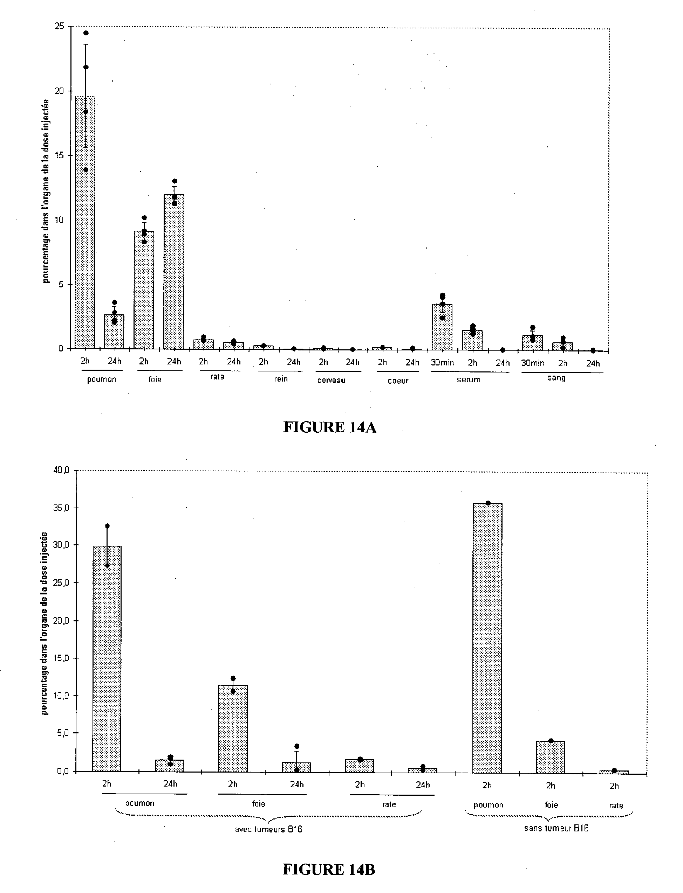

Pour chacun des deux groupes, les organes des souris ayant reçu des lipoplexes avec lipide-Rhodamine sont broyés dans du PBS (5mL par gramme d'organe), les lipides sont extraits dans 30 volumes de Chloroforme:Méthanol (1:1 v/v) et la fluorescence est dosée au Wallac Victor contre une gamme (lecteur de microplaque équipé pour le dosage de la fluorescence). Les résultats, calculés en pourcentage dans l'organe de la dose injectée, sont représentés sur la figure 14A.

Ainsi la figure 14A représente la biodistribution à 2h et 24h des lipoplexes siRNA-acide polyglutamique formulés avec du RPR:DOPE:Rhodamine dans du Glucose 5% à un ratio de charges de 8, puis injectées en intraveineuse. Les souris présentent des tumeurs B16 métastatiques dans les poumons.

Les siRNA contenus dans les organes des souris ayant reçu des lipoplexes avec siRNA-Rhodamine sont extraits selon le protocole établi avec le kit miRNeasy (Qiagen) et la fluorescence est dosée au Wallac Victor contre une gamme. Les résultats, calculés en pourcentage dans l'organe de la dose injectée, sont représentés sur la figure 14B.

Ainsi la figure 14B représente la biodistribution à 2h et 24h des lipoplexes siRNA-Rhodamine avec de l'acide polyglutamique formulés avec du RPR:DOPE dans du Glucose 5% à un ratio de charges de 8, puis injecté en intraveineuse. Les souris présentaient ou non des tumeurs B16 métastatiques dans les poumons.

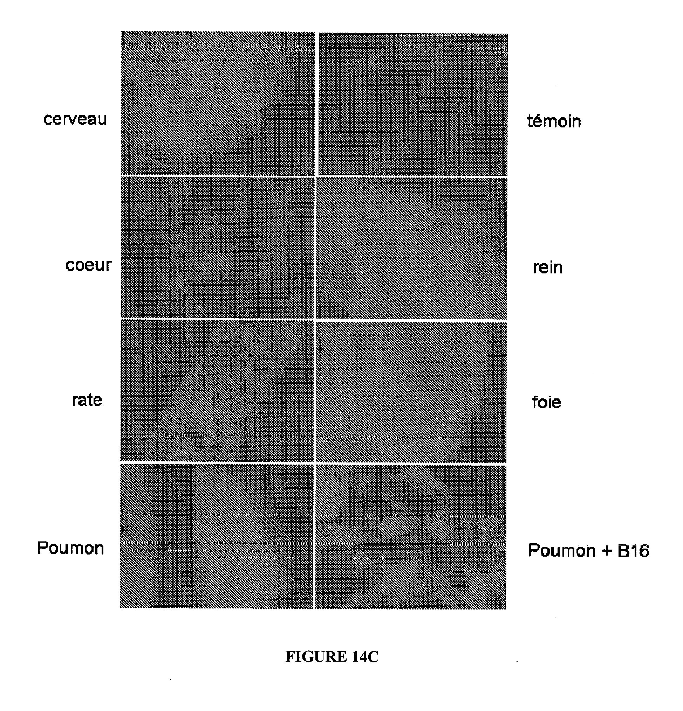

La figure 14C représente la biodistribution à 2h des lipoplexes siRNA acide polyglutamique formulées avec du RPR:DOPE:Rhodamine dans du Glucose 5% à un ratio de charges de 8, puis injectés en intraveineuse. Les souris présentaient ou non des tumeurs B16 métastatiques dans les poumons. Les témoins sont identiques pour tous les organes et ne sont donc représentés qu'une fois. Les réglages ne sont pas les mêmes pour les différents organes.

### Résultats

Comme représenté sur les figures 14A et B, une distribution est observée principalement dans les poumons à 2h (20% de la dose injectée pour les lipides et les siRNA), qui diminue très fortement après 24h. Le foie accumule environ 10% des lipides injectés comme des siRNA.

La distribution dans la rate est assez faible, environ 1-2% de la dose injectée pour les lipides et les siRNA. Les autres organes, cerveau, rein et coeur, ne renferment qu'une infime quantité de lipide-Rhodamine, probablement contenus dans les vaisseaux sanguins.

Le dosage des lipides-Rhodamine dans le sang et le sérum montrent que les lipoplexes sont principalement libres dans le sérum et peu associés à des cellules sanguines.

Sur la figure 14A, les réglages étant différents d'un organe à l'autre, il ne faut pas comparer l'intensité de la fluorescence entre les organes. Pour un même organe, l'organe transfecté et l'organe témoin ont été observés avec les mêmes réglages et les témoins ne présentaient pas de fluorescence.

Comme représenté sur la figure 14C, la fluorescence observée dans le cerveau met en relief les vaisseaux sanguins, les lipoplexes ne passant probablement pas la barrière hémato-encéphalique. La fluorescence dans le coeur et la rate est assez faible et concentrée sur certains points. Seule la fluorescence observée dans les reins, les poumons et le foie est homogène sur tout l'organe.

### Conclusion

L'ajout d'une macromolécule anionique à un siRNA avant formulation en lipoplexes avec un lipide cationique permet d'en améliorer l'efficacité d'inhibition de gène de manière significative, à la fois sur des cellules en culture mais également chez l'animal, sans entraîner de toxicité supplémentaire.

### REFERENCES BIBLIOGRAPHIQUES

PATNAIK et al., PEI-alginate nanocomposites as efficient in vitro gene transfection agents ; Journal of Control Release, 2006, 114(3):398-409
KHOURY et al., Efficient new cationic liposome formulation for systemic delivery of small interfering RNA silencing tumor necrosis factor alpha in experimental arthritis. Arthritis Rheum. 2006 Jun;54(6):1867-77.
WU et al; Let me count the ways: mechanisms of gene regulation by miRNAs and siRNAs. Mol Cell. 2008 Jan 18;29(1):1-7. Review
VAN OMMEN et al; The therapeutic potential of antisense-mediated exon skippingCurr Opin Mol Ther. 2008 Apr;10(2):140-9. Review
KAUR et ROY, Therapeutic applications of aptamers, Expert Opin Investig Drugs. 2008 Jan;17(1):43-60. Review.
Demande internationale WO 97/18185 telle que publiée le 22 mai 1997 (Rhone-Poulenc Rorer).
Brevet américain US 6,171,612 délivré le 9 janvier 2001 (Aventis Pharma).

## Revendications

1. Composition comprenant :
(1) une macromolécule anionique à l'exception des acides nucléiques,
(2) un lipide cationique, et
(3) un acide nucléique de taille inférieure ou égale à 200 nucléotides
dans laquelle ladite macromolécule anionique, ledit lipide cationique et ledit acide nucléique sont associés de manière non covalente.

2. Composition selon la revendication 1, dans laquelle la macromolécule anionique est choisie dans le groupe constitué par les polysaccharides anioniques, de préférence l'acide alginique et les polypeptides anioniques, de préférence l'acide polyglutamique.

3. Composition selon l'une quelconque des revendications 1 ou 2, **caractérisée en ce que** la macromolécule anionique a un poids moléculaire moyen compris entre 1000 et 1000000 Da, de préférence compris entre 1000 et 100000 Da, et encore plus préférentiellement compris entre 4300 et 56000 Da.

4. Composition selon l'une quelconque des revendications 1 à 3, **caractérisée en ce que** le lipide cationique est choisi parmi les lipopolyamines, telles que la 2-{3-[Bis-(3-amino-propyl)-amino]-propylamino}-N-ditetradecyl carbamoyl methyl-acetamide, la 2-{3-[3-(3-Amino-propylamino)-propylamino]-propylamino}-N,N-dioctadecyl-acetamide ou le 2,3-dioleoyloxy- N-[2(sperminecarboxamido)ethyl]-N,N-dimethyl- 1 -propane-aminium-trifluoracetate, le dioctadécylamidoglycyl spermine, la 5-carboxyspermylamide de la palmitoylphosphatidylethanolamine, les ammoniums quartenaires tels que le bromure de 1,2-dimyristyloxypropyl-3-diméthyl-hydroxy éthyl ammonium, N-[1-(2,3-dioleyloxy) propyl]-N,N,N-trimethylammonium chloride, le 1,2-_dimyristoyl-_3-_trimethylammonium propane, 1,2-dioleoyl-3-_trimethylammonium propane, la dimethyldioctadecylammonium bromide, ou la dioleoyloxypropyl dimethylhydroxyethylammonium bromide, et les lipides comportant des têtes cationiques de type guanidine ou imidazole.

5. Composition selon l'une quelconque des revendications 1 à 4, **caractérisée en ce que** le lipide cationique est formulé soit sous forme de micelles, soit sous forme de liposomes par association avec un lipide neutre tel que la dioleyl phosphatidyl éthanolamine, le Cholestérol, ou le (*N*'-(*rac*-1-[11-(*Foctyl*)undec-10-*enyl*]-2-(hexadecyl)glycero-3-phosphoethanoyl)-sperminecarboxamide).

6. Composition selon l'une quelconque des revendications 1 à 5, **caractérisée en ce que** l'acide nucléique de taille inférieure ou égale à 200 nucléotides est un acide nucléique capable de moduler une fonction protéique, tel qu'un oligonucléotide antisens, un oligonucléotide pour le saut d'exon ou un ARN interférant et/ou une forme modifiée d'un tel acide nucléique, telle que la forme morpholino, phosphorothioate, phosphoroamidate ou 2' O-méthyl.

7. Composition selon l'une quelconque des revendications 1 à 6, dans laquelle la macromolécule anionique est l'acide polyglutamique, le lipide cationique est le 2-{3-[Bis-(3-amino-propyl)-amino]-propylamino}-N-ditetradecyl carbamoyl methyl-acetamide et l'acide nucléique de taille inférieure ou égale à 200 nucléotides est un petit ARN interférant.

8. Composition selon l'une quelconque des revendications 1 à 6, dans laquelle la macromolécule anionique est l'acide alginique, le lipide cationique est le 2-{3-[Bis-(3-amino-propyl)-amino]-propylamino}-N-ditetradecyl carbamoyl methyl-acetamide et l'acide nucléique de taille inférieure ou égale à 200 nucléotides est un petit ARN interférant.

9. Procédé pour transférer *in vitro* ou *ex vivo* un acide nucléique dans des cellules, ledit procédé comprenant la mise en contact desdites cellules avec une composition selon l'une quelconque des revendications 1 à 8.

10. Utilisation d'une composition vecteur comprenant :
(1) une macromolécule anionique à l'exception des acides nucléiques, et
(2) un lipide cationique,
en tant que vecteur pharmaceutiquement acceptable d'un acide nucléique de taille inférieure ou égale à 200 nucléotides, ladite molécule anionique, ledit lipide cationique et ledit acide nucléique étant associés de manière non covalente.

11. Composition selon l'une quelconque des revendications 1 à 8 pour son utilisation en tant que médicament.

12. Composition selon la revendication 11, pour son utilisation en tant que médicament destiné au traitement d'une pathologie associée à un dysfonctionnement d'une fonction protéique.

13. Composition selon la revendication 12, dans laquelle la pathologie est choisie parmi la polyarthrite, les rhumatismes, les cancers, l'hépatite, le lupus érythémateux, la fibrose cardiaque ou hépatique, la Chorée de Huntington, l'achondroplasie, la sclérose latérale amyotrophique ou la maladie de Lou Gehrig, la myopathie de Duchenne et la polymyosite, et la maladie d'Alzheimer.

14. Procédé de préparation d'une composition telle que définie selon l'une quelconque des revendications 1 à 8, comprenant :
a) une étape de mise en contact de la macromolécule anionique avec l'acide nucléique pour la formation d'un mélange,
b) une étape de mise en contact du lipide cationique avec le mélange obtenu à l'étape a), pour la préparation de la composition.

## Patentansprüche

1. Zusammensetzung, die Folgendes umfasst:
(1) ein anionisches Makromolekül, ausgenommen von Nukleinsäuren,
(2) ein kationisches Lipid und
(3) eine Nukleinsäure mit einer Größe kleiner gleich 200 Nukleotiden,
wobei das anionische Makromolekül, das kationische Lipid und die Nukleinsäure nichtkovalent verbunden sind.

2. Zusammensetzung nach Anspruch 1, wobei das anionische Makromolekül aus der Gruppe bestehend aus anionischen Polysacchariden, vorzugsweise Alginsäure, und anionischen Polypeptiden, vorzugsweise Polyglutaminsäure, ausgewählt ist.

3. Zusammensetzung nach einem der Ansprüche 1 und 2, **dadurch gekennzeichnet, dass** das anionische Makromolekül ein durchschnittliches Molekulargewicht aufweist, das zwischen 1000 und 1.000.000 Da liegt, vorzugsweise zwischen 1000 und 100.000 Da liegt und noch mehr bevorzugt zwischen 4300 und 56.000 Da liegt.

4. Zusammensetzung nach einem der Ansprüche 1 bis 3, **dadurch gekennzeichnet, dass** das kationische Lipid aus Lipopolyaminen, wie 2-{3-[Bis-(3-aminopropyl)amino]-propylamino}-N-ditetradecylcarbamoylmethylacetamid, 2-{3-[3-(3-Aminopropylamino)propylamino]propylamino}-N,N-dioctadecylacetamid oder 2,3-Dioleoyloxy-N-[2-(spermincarboxamido)ethyl]-N,N-dimethyl-1-propanaminiumtrifluoracetat, Dioctadecylamidoglycylspermin,5-Carboxyspermylamid von Palmitoylphosphatidylethanolamin, quartären Ammoniumverbindungen, wie 1,2-Dimyristyloxypropyl-3-dimethylhydroxyethylammoniumbromid, N-[1-(2,3-Dioleyloxy)propyl]-N,N,N-trimethylammoniumchlorid, 1,2-Dimyristoyl-3-trimethylammoniumpropan, 1,2-Dioleoyl-3-trimethylammoniumpropan, Dimethyldioctadecylammoniumbromid oder Dioleoyloxypropyldimethylhydroxyethylammoniumbromid, und Lipiden, die kationische Köpfe vom Guanidin- oder Imidazoltyp umfassen, ausgewählt ist.

5. Zusammensetzung nach einem der Ansprüche 1 bis 4, **dadurch gekennzeichnet, dass** das kationische Lipid entweder in der Form von Mizellen oder in der Form von Liposomen durch Verbindung mit einem neutralen Lipid, wie Dioleylphosphatidylethanolamin, Cholesterin oder (*N*'-(*raz*.-1-[11-(*F*-Octyl)undec-10-enyl]-2-(hexadecyl)glycero-3-phosphoethanoyl)spermincarboxamid, formuliert wird.

6. Zusammensetzung nach einem der Ansprüche 1 bis 5, **dadurch gekennzeichnet, dass** die Nukleinsäure mit einer Größe kleiner gleich 200 Nukleotiden eine Nukleinsäure, die eine Proteinfunktion modulieren kann, wie ein Antisense-Oligonukleotid, ein Oligonukleotid für das Überspringen von Exons oder eine interferierende RNA, und/oder eine modifizierte Form einer derartigen Nukleinsäure ist, wie die Morpholino-, Phosphorthioat-, Phosphoramidat- oder 2'-O-Methyl-Form.

7. Zusammensetzung nach einem der Ansprüche 1 bis 6, **dadurch gekennzeichnet, dass** das anionische Makromolekül Polyglutaminsäure ist, das kationische Lipid 2-{3-[Bis-(3-aminopropyl)amino]propylamino}-N-ditetradecylcarbamoylmethylacetamid ist und die Nukleinsäure mit einer Größe kleiner gleich 200 Nukleotiden eine kurze interferierende RNA ist.

8. Zusammensetzung nach einem der Ansprüche 1 bis 6, wobei das anionische Makromolekül Alginsäure ist, das kationische Lipid 2-{3-[Bis-(3-aminopropyl)amino]-propylamino}-N-ditetradecylcarbamoylmethylacetamid ist und die Nukleinsäure mit einer Größe kleiner gleich 200 Nukleotiden eine kurze interferierende RNA ist.

9. Verfahren zum Übertragen einer Nukleinsäure in Zellen *in vitro* oder *ex vivo*, wobei das Verfahren das Inkontaktbringen der Zellen mit einer Zusammensetzung nach einem der Ansprüche 1 bis 8 umfasst.

10. Verwendung einer Vektorzusammensetzung, die Folgendes umfasst:
(1) ein anionisches Makromolekül, ausgenommen von Nukleinsäuren, und
(2) ein kationisches Lipid,
als einen pharmazeutisch unbedenklichen Vektor für eine Nukleinsäure mit einer Größe kleiner gleich 200 Nukleotiden, wobei das anionische Makromolekül, das kationische Lipid und die Nukleinsäure nichtkovalent verbunden sind.

11. Zusammensetzung nach einem der Ansprüche 1 bis 8 zu deren Verwendung als Arzneimittel.

12. Zusammensetzung nach Anspruch 11 zu deren Verwendung als Arzneimittel, das zur Behandlung einer Krankheit vorgesehen ist, die mit einer Störung einer Proteinfunktion verbunden ist.

13. Zusammensetzung nach Anspruch 12, wobei die Krankheit aus Polyarthritis, Rheumatismen, Krebserkrankungen, Hepatitis, Lupus erythematodes, kardialer oder hepatischer Fibrose, Chorea Huntington, Achondroplasie, amyotrophischer Lateralsklerose oder Lou-Gehrig-Syndrom, Duchenne-Muskeldystrophie und Polymyositis und Alzheimer-Krankheit ausgewählt ist.

14. Verfahren zur Herstellung einer wie in einem der Ansprüche 1 bis 8 definierten Zusammensetzung, wobei das Verfahren Folgendes umfasst:
a) einen Schritt des Inkontaktbringens des anionischen Makromoleküls mit der Nukleinsäure zur Bildung eines Gemischs,
b) einen Schritt des Inkontaktbringens des kationischen Lipids mit dem in Schritt a) erhaltenen Gemisch zur Herstellung der Zusammensetzung.

## Claims

1. Composition comprising:
(1) an anionic macromolecule except for nucleic acids,
(2) a cationic lipid, and
(3) a nucleic acid of size less than or equal to 200 nucleotides
wherein said anionic macromolecule, said cationic lipid and said nucleic acid are noncovalently associated.

2. Composition according to claim 1, wherein the anionic macromolecule is selected from the group consisting of anionic polysaccharides, preferably alginic acid, and anionic polypeptides, preferably polyglutamic acid.

3. Composition according to any one of claims 1 or 2, **characterized in that** the anionic macromolecule has an average molecular weight comprised between 1,000 and 1,000,000 Da, preferably comprised between 1,000 and 100,000 Da, and even more preferentially comprised between 4,300 and 56,000 Da.

4. Composition according to any one of claims 1 to 3, **characterized in that** the cationic lipid is selected among lipopolyamines, such as 2-{3-[bis-(3-amino-propyl)-amino]-propylamino}-N-ditetradecyl carbamoyl methyl-acetamide, 2-{3-[3-(3-amino-propylamino)-propylamino]-propylamino}-N,N-dioctadecyl-acetamide or 2,3-dioleoyloxy-N-[2(sperminecarboxamido)ethyl]-N,N-dimethyl-1-propaneaminium-trifluoracetate, dioctadecylamidoglycyl spermine, palmitoylphosphatidylethanolamine 5-carboxyspermylamide, quaternary ammoniums such as 1,2-dimyristyloxypropyl-3-dimethyl-hydroxy ethyl ammonium bromide, N-[1-(2,3-dioleyloxy)propyl]-N,N,N-trimethylammonium chloride, 1,2-dimyristoyl-3-trimethylammonium propane, 1,2-dioleoyl-3-trimethylammonium propane, dimethyldioctadecylammonium bromide, or dioleoyloxypropyl dimethylhydroxyethylammonium bromide, and lipids comprising guanidine or imidazole cationic heads.

5. Composition according to any one of claims 1 to 4, **characterized in that** the cationic lipid is formulated either in the form of micelles, or in the form of liposomes by association with a neutral lipid such as dioleyl phosphatidyl ethanolamine, cholesterol, or (N'-(rac-1-[11-(F-octyl)undec-10-enyl]-2-(hexadecyl)glycero-3-phosphoethanoyl)-sperminecarboxamide).

6. Composition according to any one of claims 1 to 5, **characterized in that** the nucleic acid of size less than or equal to 200 nucleotides is a nucleic acid able to modulate a protein function, such as an antisense oligonucleotide, an oligonucleotide for exon skipping or an interfering RNA and/or a modified form of one such nucleic acid, such as the morpholino, phosphorothioate, phosphoroamidate or 2' O-methyl form.

7. Composition according to any one of claims 1 to 6, wherein the anionic macromolecule is polyglutamic acid, the cationic lipid is 2-{3-[bis-(3-amino-propyl)-amino]-propylamino}-N-ditetradecyl carbamoyl methyl-acetamide and the nucleic acid of size less than or equal to 200 nucleotides is a small interfering RNA.

8. Composition according to any one of claims 1 to 6, wherein the anionic macromolecule is alginic acid, the cationic lipid is 2-{3-[bis-(3-amino-propyl)-amino]-propylamino}-N-ditetradecyl carbamoyl methyl-acetamide and the nucleic acid of size less than or equal to 200 nucleotides is a small interfering RNA.

9. Method for transferring *in vitro* or *ex vivo* a nucleic acid in cells, said method comprising the contacting of said cells with a composition according to any one of claims 1 to 8.

10. Use of a vector composition comprising:
(1) an anionic macromolecule except for nucleic acids, and
(2) a cationic lipid,
as a pharmaceutically acceptable vector for a nucleic acid of size less than or equal to 200 nucleotides, said anionic macromolecule, said cationic lipid and said nucleic acid being noncovalently associated.

11. Composition according to any one of claims 1 to 8 for its use as a drug.

12. Composition according to claim 11, for its use as a drug intended for the treatment of a pathology associated with a dysfunction of a protein function.

13. Composition according to claim 12, wherein the pathology is selected from polyarthritis, rheumatism, cancer, hepatitis, lupus erythematosus, cardiac or hepatic fibrosis, Huntington's chorea, achondroplasia, amyotrophic lateral sclerosis or Lou Gehrig's disease, Duchenne muscular dystrophy and polymyositis, and Alzheimer's disease.

14. Method for preparing a composition as defined in any one of claims 1 to 8, comprising:
a) a step of contacting the anionic macromolecule with the nucleic acid for the formation of a mixture,
b) a step of contacting the cationic lipid with the mixture obtained in step a), for the preparation of the composition.
